# EUROPÄISCHE PATENTANMELDUNG

(11) **EP 2 848 228 A1**
(43) Veröffentlichungstag der Anmeldung: **18.03.2015**
(21) Anmeldenummer: 13184654.5
(22) Anmeldetag: 16.09.2013
(51) Int. Cl.: A61B 19/02, A61B 17/00, A61B 18/00

(54) **Solare Aufladetechnik für ein steril verpacktes Medizinprodukt**

(71) Anmelder: Corlife oHG, 30625 Hannover (DE)
(72) Erfinder: Die Erfindernennung liegt noch nicht vor
(74) Vertreter: Arth, Hans-Lothar

(57) **Zusammenfassung**

Die vorliegende Erfindung betrifft ein steril verpacktes sterilisiertes Medizinprodukt mit photovoltaischem Konverter und einer internen Energieversorgungseinheit, welche sich im steril verpackten Zustand über den photovoltaischen Konverter aufladen lässt sowie eine Aufladevorrichtung und ein Verfahren zum Aufladen der internen Energieversorgungseinheit eines steril verpackten sterilisierten Medizinproduktes.

## Beschreibung

Die vorliegende Erfindung betrifft ein steril verpacktes Medizinprodukt mit photovoltaischem Konverter, insbesondere Solarzelle oder Photodiode, und einer internen Energieversorgungseinheit, welche sich im steril verpackten Zustand über den photovoltaischen Konverter aufladen lässt, sowie eine Aufladevorrichtung und ein Verfahren zum Aufladen der internen Energieversorgungseinheit eines steril verpackten Medizinproduktes.

In der Medizintechnik werden immer häufiger Geräte und Instrumente mit elektrisch betriebenen Komponenten verwendet. Dies reicht von einfachen chirurgischen Werkzeugen wie z.B. beleuchtete Wundhaken bis hin zu komplexen elektronischen Implantaten wie z.B. Herzschrittmachern oder Cochlea-Implantaten. Hierbei wird zunehmend auf Medizinprodukte mit interner Energieversorgung z.B. durch Batterien (auch Primärzelle oder Primärelement) oder wiederaufladbare Batterien (Akkumulator, Akku oder auch Sekundärzelle bzw. Sekundärelement) gesetzt. So bieten chirurgische Instrumente mit eingebautem Akkumulator eine deutlich höhere Bewegungsfreiheit für die bedienende Person als Instrumente mit einem Kabel zur Stromversorgung. Berichten zufolge wird für den Bereich der mikroelektronischen Implantate ein jährliches Marktwachstum von fast 9 % bis zum Jahr 2016 vorausgesagt.

Bei der Verwendung von Geräten mit interner Energieversorgung im medizinischen Bereich ist es von essentieller Bedeutung, dass zum Zeitpunkt der Verwendung die höchstmögliche Energieversorgung gewährleistet ist, d.h. dass ein verbauter Akku vollständig aufgeladen ist. Sollte dies nicht der Fall sein, kann dies zu einem Ausfall des Gerätes und somit im schlimmsten Fall zu ernsthaften gesundheitlichen wenn nicht gar lebensbedrohlichen Konsequenzen für den Patienten führen. Aus diesem Grund hat auch die MHRA (Medicines and Healthcare Products RegulatoryAgency), eine Abteildung des Gesundheitsministeriums in Großbritannien, im Jahr 2005 einen ausführlichen Leitfaden zum Umgang mit elektrisch betriebenen medizinischen Geräten herausgebracht, in dem detailliert auf die Probleme und Konsequenzen nicht vollständig geladener medizinischer Geräte eingegangen wird.

Dieses Problem betrifft allerdings nicht nur wiederverwendbare Produkte sondern auch Produkte, die für den Einmalgebrauch vorgesehen sind. Ursächlich hierfür ist das bei Batterien und Akkus auftretende Phänomen der Selbstentladung. Die Selbstentladung bezeichnet einen Kapazitätsverlust der Batterie bzw. des Akkus, der eigenständig d.h. ohne den Anschluss eines Energieabnehmers stattfindet. Dieser Prozess ist abhängig von der Dauer der Lagerung, den Lagerungsbedingungen (z.B. Temperatur) und vom Typ der Batterie bzw. des Akkus. Folglich können gerade längere Lagerungszeiten zwischen Herstellung und Verwendung des Medizinproduktes sowie unterschiedliche Temperaturbedingungen (z.B. während des Transportes) zu einer unerwünschten Entladung der Batterie bzw. des Akkus führen. Hinzu kommt, dass die angesprochenen Medizinprodukte in der Regel steril sein müssen und dies auch bis zu der Verwendung am Patienten bleiben müssen. Dies führt zu zwei Problemen: Zum einen wird durch den Sterilisationsprozess, für den zumeist erhöhte Temperaturen notwendig sind, der Prozess der Selbstentladung gefördert. Zum anderen ist es nicht zweckdienlich im Sinne der Sterilität des Produktes, dieses vor der Verwendung der Sterilverpackung zu entnehmen, um den verbauten Akku erneut laden zu können. Hinzu kommt, dass für viele Akkus und besonders für Lithium-Ionen-Akkus, die wegen ihrer vergleichsweise hohen Energiedichte häufig für Medizinprodukte verwendet werden, bei einer längeren Lagerung empfohlen wird, die Lagerung nicht im vollständig geladenen Zustand durchzuführen um die Lebensdauer der Akkus zu verlängern.

Aufgabe der vorliegenden Erfindung ist es daher, eine Möglichkeit bereitzustellen, in einer Sterilverpackung befindliche Medizinprodukte mit einer internen Energieversorgungseinheit nach der Sterilisation aufzuladen, ohne dabei die Sterilität der Sterilverpackung bzw. des Medizinproduktes zu beeinflussen oder zu gefährden.

Diese Aufgabe wird erfindungsgemäß durch die technische Lehre der unabhängigen Ansprüche gelöst. Weitere vorteilhafte Ausgestaltungen der Erfindung ergeben sich aus den abhängigen Ansprüchen, der Beschreibung, den Figuren sowie den Beispielen.

Die Aufgabe wird durch ein Medizinprodukt gelöst, welches mindestens einen photovoltaischen Konverter zum Aufladen der internen Energieversorgungseinheit aufweist und die Bestrahlung des mindestens einen photovoltaischen Konverters durch die Sterilverpackung hindurch erfolgen kann, ohne die Sterilität zu mindern oder das Medizinprodukt zu kontaminieren.

Daher betrifft die vorliegende Erfindung ein in einer Sterilverpackung befindliches sterilisiertes Medizinprodukt mit einer internen Energieversorgungseinheit und mindestens einem auf, an oder in der Oberfläche des Medizinproduktes befindlichen photovoltaischen Konverter, wobei die interne Energieversorgungseinheit durch den mindestens einen photovoltaischen Konverter aufladbar ist und die Sterilverpackung zumindest in dem den mindestens einen photovoltaischen Konverter bedeckenden Bereich durchlässig für Strahlung zur Erzeugung von elektrischem Strom mittels des mindestens einen photovoltaischen Konverters ist.

Der Begriff **"photovoltaischer Konverter",** wie hierin verwendet, bezeichnet ein Bauteil, welches die Umwandlung von Lichtenergie in elektrische Energie durch Ausnutzung des photoelektrischen Effektes ermöglicht und umfasst somit sowohl Solarzellen als auch Photodioden.

Daher betrifft die vorliegende Erfindung ein in einer Sterilverpackung befindliches sterilisiertes Medizinprodukt mit einer internen Energieversorgungseinheit und mindestens einer auf, an oder in der Oberfläche des Medizinproduktes befindlichen Solarzelle, wobei die interne Energieversorgungseinheit durch die mindestens eine Solarzelle aufladbar ist und die Sterilverpackung zumindest in dem die mindestens eine Solarzelle bedeckenden Bereich durchlässig für Strahlung zur Erzeugung von elektrischem Strom mittels der mindestens einen Solarzelle ist.

Daher betrifft die vorliegende Erfindung ein in einer Sterilverpackung befindliches sterilisiertes Medizinprodukt mit einer internen Energieversorgungseinheit und mindestens einer auf, an oder in der Oberfläche des Medizinproduktes befindlichen Photodiode, wobei die interne Energieversorgungseinheit durch die mindestens eine Photodiode aufladbar ist und die Sterilverpackung zumindest in dem die mindestens eine Photodiode bedeckenden Bereich durchlässig für Strahlung zur Erzeugung von elektrischem Strom mittels der mindestens einen Photodiode ist.

Der Begriff **"Strahlung",** wie hierin verwendet, bezieht sich vorzugsweise auf elektromagnetische Strahlung.

Grundgedanke der vorliegenden Erfindung ist die Bereitstellung eines sterilen Medizinproduktes, dessen interne Energieversorgungseinheit genau zum Zeitpunkt der Verwendung vollständig aufgeladen ist, ohne dass durch den Aufladevorgang die Sterilität von Produkt und Verpackung beeinträchtigt wird. Hierzu wird ein zunächst unsteriles Medizinprodukt, welches eine interne Energieversorgungseinheit, vorzugsweise einen Akku, sowie zumindest einen auf, an oder in der Oberfläche des Medizinproduktes befindlichen photovoltaischen Konverter, insbesondere Solarzelle oder Photodiode, enthält in einer zur Sterilisation geeigneten Verpackung (z.B. in Gestalt eines Blisters) verpackt und die Verpackung wird versiegelt. Anschließend wird die zur Sterilisation geeignete Verpackung samt Medizinprodukt durch die Verpackung hindurch sterilisiert. Vor der Auslieferung oder einige Zeit vor der Verwendung des Medizinproduktes besteht die Notwendigkeit, die interne Energieversorgungseinheit vollständig aufzuladen, weil sich die interne Energieversorgungseinheit während der Lagerung teilweise entlädt oder vor der Sterilisation nicht vollständig aufgeladen worden war. Die vollständige Aufladung der internen Energieversorgungseinheit erfolgt nun durch Bestrahlung des photovoltaischen Konverters mittels externer Lichtquelle durch die Sterilverpackung hindurch, ohne die Sterilität zu mindern oder das Medizinprodukt zu kontaminieren.

In der Regel genügt ein photovoltaischer Konverter für ein Medizinprodukt, so dass die vorliegende Erfindung bevorzugt gerichtet ist auf ein in einer Sterilverpackung befindliches sterilisiertes Medizinprodukt mit einer internen Energieversorgungseinheit und einem auf, an oder in der Oberfläche des Medizinproduktes befindlichen photovoltaischen Konverter, wobei die interne Energieversorgungseinheit durch den photovoltaischen Konverter aufladbar ist und die Sterilverpackung zumindest in dem den photovoltaischen Konverter bedeckenden Bereich durchlässig für Strahlung zur Erzeugung von elektrischem Strom mittels des photovoltaischen Konverters ist.

In der Regel genügt eine Solarzelle für ein Medizinprodukt, so dass die vorliegende Erfindung bevorzugt gerichtet ist auf ein in einer Sterilverpackung befindliches sterilisiertes Medizinprodukt mit einer internen Energieversorgungseinheit und einer auf, an oder in der Oberfläche des Medizinproduktes befindlichen Solarzelle, wobei die interne Energieversorgungseinheit durch die Solarzelle aufladbar ist und die Sterilverpackung zumindest in dem die Solarzelle bedeckenden Bereich durchlässig für Strahlung zur Erzeugung von elektrischem Strom mittels der Solarzelle ist.

In der Regel genügt eine Photodiode für ein Medizinprodukt, so dass die vorliegende Erfindung bevorzugt gerichtet ist auf ein in einer Sterilverpackung befindliches sterilisiertes Medizinprodukt mit einer internen Energieversorgungseinheit und einer auf, an oder in der Oberfläche des Medizinproduktes befindlichen Photodiode, wobei die interne Energieversorgungseinheit durch die Photodiode aufladbar ist und die Sterilverpackung zumindest in dem die Photodiode bedeckenden Bereich durchlässig für Strahlung zur Erzeugung von elektrischem Strom mittels der Photodiode ist.

Der Begriff **"Medizinprodukt"** wie hierin verwendet betrifft sämtliche in der Medizin eingesetzten medizinischen Geräte, Instrumente und Vorrichtungen sowie Teile davon, welche eine interne Energieversorgungseinheit zur Lieferung von Strom besitzen und welche steril verpackt sein müssen. Die interne Energieversorgungseinheit liefert dabei Strom vorzugsweise zur Versorgung einer Lichtquelle, eines Motors oder eines Impulsgebers. Der Stromverbraucher befindet sich dabei vorzugsweise auch im Medizinprodukt selbst. Es ist aber auch möglich, dass der Strom von der internen Energieversorgungseinheit für einen Stromverbraucher bereitgestellt wird, der sich außerhalb des Medizinproduktes selbst befindet. Dies ist vor allem dann der Fall, wenn das Medizinprodukt ein Teil eines medizinischen Gerätes, medizinischen Instrumentes oder einer medizinischen Vorrichtung ist.

Beispiele für erfindungsgemäß einsetzbare Medizinprodukte sind Beleuchtungsvorrichtungen, insbesondere für die Ausleuchtung eines Operationssitus, Defibrillatoren, Herzschrittmacher, Cochlea-Implantate, Sensoren für z.B. Druck und/oder Temperatur, Elektrokauter, Ultraschallskalpelle, und bestimmte kardiovaskuläre, neurologische oder orthopädische Implantate, sofern diese über eine elektronische Komponente besitzen. Ferner kann es sich um Implantate zur Wirkstofffreisetzung (z.B. Piezopumpen) handeln.

Ist das erfindungsgemäß einsetzbare Medizinprodukt hingegen Teil eines medizinischen Gerätes, eines medizinischen Instrumentes oder einer medizinischen Vorrichtung, so kann es sich bei dem medizinischen Gerät, dem medizinischen Instrument oder der medizinischen Vorrichtung zum Beispiel um Instrumente zum Spreizen und Halten (z.B. Retraktoren), Spekula, Pinzetten, Klemmen, Clips, Skalpelle, Scheren, Stanzen, Meißel, Raspatorien, Küretten, Fräsen, Stößel, Löffel, Sauger, Bohrer, Rohrschaftinstrumente (z.B. Trokare), Zangen handeln.

In einer bevorzugten Ausführungsform der vorliegenden Erfindung handelt es sich bei dem Medizinprodukt um Cochlea-Implantate, Herzschrittmacher, Herzunterstützungssysteme, Defibrillatoren, neurologische Implantate, Kunstsphinkter mit elektronischer Komponente, elektrisch betriebene orthopädische Implantate (z.B. Marknagel der über einen integrierten Elektromotor auseinander gefahren wird um die Callus-Bildung zu fördern), Sensoren (z.B. für Druck und/oder Temperatur), abnehmbare Beleuchtungsvorrichtungen für chirurgische Instrumente (wie z.B. Sperrer- und Halteblätter von Retraktoren und Spreizsystemen), chirurgische Instrumente mit fest integrierter Beleuchtungsvorrichtung, elektrische Einmalprodukte, elektrische Komponenten für die Roboter-gestützte Chirurgie, Wirkstoff-freisetzende Implantate und dergleichen.

Die vorliegende Erfindung betrifft ein in einer Sterilverpackung befindliches sterilisiertes Medizinprodukt mit einer internen Energieversorgungseinheit und mindestens einem auf, an oder in der Oberfläche des Medizinproduktes befindlichen photovoltaischen Konverter, wobei die interne Energieversorgungseinheit durch den mindestens einen photovoltaischen Konverter aufladbar ist und die Sterilverpackung zumindest in dem den mindestens einen photovoltaischen Konverter bedeckenden Bereich durchlässig für elektromagnetische Strahlung zur Erzeugung von elektrischem Strom mittels des mindestens einen photovoltaischen Konverters ist.

Die vorliegende Erfindung betrifft ein in einer Sterilverpackung befindliches sterilisiertes Medizinprodukt mit einer internen Energieversorgungseinheit und mindestens einer auf, an oder in der Oberfläche des Medizinproduktes befindlichen Solarzelle, wobei die interne Energieversorgungseinheit durch die mindestens eine Solarzelle aufladbar ist und die Sterilverpackung zumindest in dem die mindestens eine Solarzelle bedeckenden Bereich durchlässig für elektromagnetische Strahlung zur Erzeugung von elektrischem Strom mittels der mindestens einen Solarzelle ist.

Die vorliegende Erfindung betrifft ein in einer Sterilverpackung befindliches sterilisiertes Medizinprodukt mit einer internen Energieversorgungseinheit und mindestens einer auf, an oder in der Oberfläche des Medizinproduktes befindlichen Photodiode, wobei die interne Energieversorgungseinheit durch die mindestens eine Photodiode aufladbar ist und die Sterilverpackung zumindest in dem die mindestens eine Photodiode bedeckenden Bereich durchlässig für elektromagnetische Strahlung zur Erzeugung von elektrischem Strom mittels der mindestens einen Photodiode ist.

Die in der vorliegenden Erfindung verwendeten Medizinprodukte sind ferner dadurch charakterisiert, dass sie eine interne Energieversorgungseinheit besitzen. In einer bevorzugten Ausführungsform der vorliegenden Erfindung, handelt es sich bei der internen Energieversorgungseinheit um einen **Akkumulator** (Akku, auch Sekundärzelle oder Sekundärelement). Beispiele für Akkumulatoren sind: Nickel-Cadmium-Akku (NiCd), Nickel-Metallhydrid-Akku (NiMH), Lithium-Ionen-Akkus (Li-Ion) u.a. umfassend Lithium-Cobaltdioxid-Akku (LiCoO₂), Lithium-Polymer-Akku (LiPo), Lithium-Mangan-Akku (LiMn), Lithium-Eisenphosphat-Akku (LiFePO₄), Lithium-Schwefel-Akku (LiS), sowie Lithium-Titanat-Akku (LiTi). Dem Fachmann sind weitere Ausführungsformen von Akkus bekannt, die in der vorliegenden Erfindung verwendet werden können.

Die in einer Sterilverpackung befindlichen Medizinprodukte sind mit einer internen Energieversorgungseinheit und mindestens einem auf, an oder in der Oberfläche des Medizinproduktes befindlichen **photovoltaischen Konverter versehen.** Der photovoltaische Konverter ist vorzugsweise eine Solarzelle, die bevorzugt aus der folgenden Gruppe ausgewählt wird: monokristalline Siliziumzellen (c-Si), polykristalline oder multikristalline Siliziumzellen (poly-Si oder mc-Si), Zellen aus amorphem Silizium (a-Si), Zellen aus mikrokristallinem Silizium (µc-Si), Gallium-Arsenid-Zellen (GaAs), Cadmium-Tellurid-Zellen (CdTe), Kupfer-Indium-(Disulfid/Diselenid)-Zellen (CIS), , Kupfer-Gallium-Diselenid-Zellen (CGS), Kupfer-Indium-Gallium-(Disulfid/Diselenid)-Zellen (CIGS), elektrochemische Farbstoff-Solarzellen (auch Grätzel-Zelle), organische Solarzellen, Hybrid-Solarzellen oder einer Kombination vorstehender Zelltypen. Dem Fachmann sind weitere Ausführungsformen von Solarzellen bekannt, die in der vorliegenden Erfindung verwendet werden können. Ist der zumindest eine photovoltaische Konverter als Photodiode ausgebildet, so wird diese vorzugsweise aus folgender Gruppe ausgewählt: Silizium-Diode, Germanium-Diode.

Erfindungsgemäß kann sich der eine oder der mindestens eine photovoltaische Konverter auf der Oberfläche des Medizinproduktes oder in der Oberfläche des Medizinproduktes oder an der Oberfläche des Medizinproduktes, d.h. in Nähe des Medizinproduktes, befinden.

Erfindungsgemäß kann sich die eine oder die mindestens eine Solarzelle auf der Oberfläche des Medizinproduktes oder in der Oberfläche des Medizinproduktes oder an der Oberfläche des Medizinproduktes, d.h. in Nähe des Medizinproduktes, befinden.

Erfindungsgemäß kann sich die eine oder die mindestens eine Photodiode auf der Oberfläche des Medizinproduktes oder in der Oberfläche des Medizinproduktes oder an der Oberfläche des Medizinproduktes, d.h. in Nähe des Medizinproduktes, befinden.

Der Begriff **"auf** der Oberfläche des Medizinproduktes" wie hierin verwendet, bedeutet, dass der mindestens eine photovoltaische Konverter, insbesondere Solarzelle oder Photodiode, in direktem Kontakt mit der Oberfläche des Medizinproduktes steht und flächig mit dieser verbunden ist jedoch nicht von einer mit dem Medizinprodukt gemeinsamen Schicht oder Hülle oder Gehäuse überzogen ist, wobei es sich bei dieser Schicht oder Hülle oder Gehäuse nicht um die Sterilverpackung handelt. Hierbei ist der photovoltaische Konverter erhaben auf der Oberfläche des Medizinproduktes angebracht.

Der Begriff **"in** der Oberfläche des Medizinproduktes" wie hierin verwendet, bedeutet, dass der mindestens eine photovoltaische Konverter, insbesondere Solarzelle oder Photodiode, in die Oberfläche des Medizinproduktes integriert ist, wobei der photovoltaische Konverter in eine entsprechende Aussparung der Oberfläche eingelassen ist und somit bündig mit dieser abschließt, in die Oberfläche eingeschweißt ist und/oder von zumindest einer mit dem Medizinprodukt gemeinsamen Schicht oder Hülle oder Gehäuse (z.B. einer Materialschicht des Gehäuses des Medizinproduktes) umgeben oder bedeckt ist, wobei es sich bei dieser Schicht oder Hülle oder Gehäuse nicht um die Sterilverpackung handelt. Hierbei ist jegliche nach außen weisende und den photovoltaischen Konverter bedeckende Schicht dadurch charakterisiert, dass sie eine mittlere Durchlässigkeit (Transmission) für Strahlung der Wellenlänge von bevorzugt 300 nm bis 1100 nm von ≥ 50 %, weiterhin bevorzugt von ≥60 %, noch weiter bevorzugt von ≥ 70%, weiterhin bevorzugt von ≥ 75%, noch weiter bevorzugt von ≥ 80% und am meisten bevorzugt von ≥ 85% aufweist. Des Weiteren ist bevorzugt, wenn jegliche nach außen weisende und den photovoltaischen Konverter bedeckende Schicht dadurch charakterisiert ist, dass sie eine Dicke von ≤ 3 mm, weiter bevorzugt ≤ 1 mm, noch weiter bevorzugt ≤ 0,5 mm aufweist.

Der Begriff **"an** der Oberfläche des Medizinproduktes", wie hierin verwendet, bedeutet, dass der mindestens eine photovoltaische Konverter, insbesondere Solarzelle oder Photodiode, sich in räumlicher Nähe zu der Oberfläche des Medizinproduktes befindet, jedoch - abgesehen von einem Stromleiter - nicht direkt mit dem Medizinprodukt verbunden ist. In einer bevorzugten Ausführungsform der vorliegenden Erfindung ist der an der Oberfläche des Medizinproduktes befindliche photovoltaische Konverter über ein stromleitendes Element oder nur über ein stromleitendes Element mit dem Medizinprodukt verbunden. Weiterhin ist bevorzugt, dass diese in Form eines stromleitenden Elementes realisierte Verbindung des an der Oberfläche des Medizinproduktes befindlichen photovoltaischen Konverters mit dem Medizinprodukt abtrennbar ausgestaltet ist. In einer weiteren bevorzugten Ausführungsform der vorliegenden Erfindung ist die an der Oberfläche des Medizinproduktes befindliche Solarzelle über ein stromleitendes Element oder nur über ein stromleitendes Element mit dem Medizinprodukt verbunden. Weiterhin ist bevorzugt, dass diese in Form eines stromleitenden Elementes realisierte Verbindung der an der Oberfläche des Medizinproduktes befindlichen Solarzelle mit dem Medizinprodukt abtrennbar ausgestaltet ist. In einer weiteren bevorzugten Ausführungsform der vorliegenden Erfindung ist die an der Oberfläche des Medizinproduktes befindliche Photodiode über ein stromleitendes Element oder nur über ein stromleitendes Element mit dem Medizinprodukt verbunden. Weiterhin ist bevorzugt, dass diese in Form eines stromleitenden Elementes realisierte Verbindung der an der Oberfläche des Medizinproduktes befindlichen Photodiode mit dem Medizinprodukt abtrennbar ausgestaltet ist.

Eine weitere bevorzugte Ausführungsform der vorliegenden Erfindung betrifft daher ein erfindungsgemäßes Medizinprodukt, wobei der mindestens eine photovoltaische Konverter stromleitend und abtrennbar mit dem Medizinprodukt verbunden ist.

Eine weitere bevorzugte Ausführungsform der vorliegenden Erfindung betrifft daher ein erfindungsgemäßes Medizinprodukt, wobei die mindestens eine Solarzelle stromleitend und abtrennbar mit dem Medizinprodukt verbunden ist.

Eine weitere bevorzugte Ausführungsform der vorliegenden Erfindung betrifft daher ein erfindungsgemäßes Medizinprodukt, wobei die mindestens eine Photodiode stromleitend und abtrennbar mit dem Medizinprodukt verbunden ist.

Hierbei ist natürlich denkbar, dass die Abtrennung des photovoltaischen Konverters, insbesondere Solarzelle oder Photodiode, der Verringerung der Größe des Medizinproduktes dient, das letztlich am oder im Patienten verwendet wird (z.B. im Fall von Implantaten). Eine bevorzugte Ausführungsform der vorliegenden Erfindung betrifft daher ein in einer Sterilverpackung befindliches sterilisiertes Medizinprodukt mit einer internen Energieversorgungseinheit und mindestens einem an oder neben der Oberfläche des Medizinproduktes befindlichen photovoltaischen Konverter, wobei die interne Energieversorgungseinheit durch den mindestens einen photovoltaischen Konverter aufladbar ist und die Sterilverpackung zumindest in dem den mindestens einen photovoltaischen Konverter bedeckenden Bereich durchlässig für Strahlung zur Erzeugung von elektrischem Strom mittels des mindestens einen photovoltaischen Konverters ist und der mindestens eine photovoltaische Konverter stromleitend und abtrennbar mit dem Medizinprodukt verbunden ist. Eine weitere bevorzugte Ausführungsform der vorliegenden Erfindung betrifft daher ein in einer Sterilverpackung befindliches sterilisiertes Medizinprodukt mit einer internen Energieversorgungseinheit und mindestens einer an oder neben der Oberfläche des Medizinproduktes befindlichen Solarzelle, wobei die interne Energieversorgungseinheit durch die mindestens eine Solarzelle aufladbar ist und die Sterilverpackung zumindest in dem die mindestens eine Solarzelle bedeckenden Bereich durchlässig für Strahlung zur Erzeugung von elektrischem Strom mittels der mindestens einen Solarzelle ist und die mindestens eine Solarzelle stromleitend und abtrennbar mit dem Medizinprodukt verbunden ist. Eine weitere bevorzugte Ausführungsform der vorliegenden Erfindung betrifft daher ein in einer Sterilverpackung befindliches sterilisiertes Medizinprodukt mit einer internen Energieversorgungseinheit und mindestens einer an oder neben der Oberfläche des Medizinproduktes befindlichen Photodiode, wobei die interne Energieversorgungseinheit durch die mindestens eine Photodiode aufladbar ist und die Sterilverpackung zumindest in dem die mindestens eine Photodiode bedeckenden Bereich durchlässig für Strahlung zur Erzeugung von elektrischem Strom mittels der mindestens einen Photodiode ist und die mindestens eine Photodiode stromleitend und abtrennbar mit dem Medizinprodukt verbunden ist.

Weiter bevorzugt betrifft die vorliegende Erfindung ein in einer Sterilverpackung befindliches sterilisiertes Medizinprodukt mit einer internen Energieversorgungseinheit und mindestens einem an oder neben der Oberfläche des Medizinproduktes befindlichen photovoltaischen Konverter, wobei die interne Energieversorgungseinheit durch den mindestens einen photovoltaischen Konverter aufladbar ist und die Sterilverpackung zumindest in dem den mindestens einen photovoltaischen Konverter bedeckenden Bereich eine mittlere Durchlässigkeit für Strahlung der Wellenlänge von 300 nm bis 1100 nm von mindestens 50 %, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % besitzt und der mindestens eine photovoltaische Konverter stromleitend und abtrennbar mit dem Medizinprodukt verbunden ist.

Weiter bevorzugt betrifft die vorliegende Erfindung ein in einer Sterilverpackung befindliches sterilisiertes Medizinprodukt mit einer internen Energieversorgungseinheit und mindestens einer an oder neben der Oberfläche des Medizinproduktes befindlichen Solarzelle, wobei die interne Energieversorgungseinheit durch die mindestens eine Solarzelle aufladbar ist und die Sterilverpackung zumindest in dem die mindestens eine Solarzelle bedeckenden Bereich eine mittlere Durchlässigkeit für Strahlung der Wellenlänge von 300 nm bis 1100 nm von mindestens 50 %, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % besitzt und die mindestens eine Solarzelle stromleitend und abtrennbar mit dem Medizinprodukt verbunden ist.

Weiter bevorzugt betrifft die vorliegende Erfindung ein in einer Sterilverpackung befindliches sterilisiertes Medizinprodukt mit einer internen Energieversorgungseinheit und mindestens einer an oder neben der Oberfläche des Medizinproduktes befindlichen Photodiode, wobei die interne Energieversorgungseinheit durch die mindestens eine Photodiode aufladbar ist und die Sterilverpackung zumindest in dem die mindestens eine Photodiode bedeckenden Bereich eine mittlere Durchlässigkeit für Strahlung der Wellenlänge von 300 nm bis 1100 nm von mindestens 50 %, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % besitzt und die mindestens eine Photodiode stromleitend und abtrennbar mit dem Medizinprodukt verbunden ist.

Diese Ausführungsform ist insbesondere für kleine Medizinprodukte (z.B. Herzschrittmacher) vorteilhaft, welche nicht groß genug sind, um einen photovoltaischen Konverter, insbesondere Solarzelle oder Photodiode, zu integrieren oder anzubringen. Erfindungsgemäß befindet sich dann der mindestens eine photovoltaische Konverter an oder neben dem Medizinprodukt innerhalb der Sterilverpackung. Der mindestens eine photovoltaische Konverter ermöglicht weiterhin die Aufladung der internen Energieversorgungseinheit des Medizinproduktes mittels Strahlung durch die Sterilverpackung hindurch. Nach der Entnahme des Medizinproduktes aus der Sterilverpackung wird dann der photovoltaische Konverter zusammen mit oder ohne das verbindende stromleitende Element, beispielsweise ein Stromkabel, abgetrennt. Der am Medizinprodukt befindliche Anschluss (z.B. Stecker) für die stromleitende Verbindung zum mindestens einen photovoltaischen Konverter kann versiegelt oder steril verschlossen werden oder als Anschluss zur Verbindung mit einem medizinischen Gerät oder Instrument dienen, welches Strom benötigt und durch die interne Energieversorgungseinheit des Medizinproduktes gespeist wird.

Damit die interne Energieversorgungseinheit, vorzugsweise in Form eines Akkus, des erfindungsgemäßen Medizinproduktes durch den mindestens einen photovoltaischen Konverter, insbesondere Solarzelle oder Photodiode, aufgeladen werden kann, ist in einer bevorzugten Ausführungsform des erfindungsgemäßen Medizinproduktes in der internen Elektronik zudem ein Aufwärtswandler (auch Spannungsaufwärtswandler, Step-Up-Wandler, Hochsetzsteller, Aufwärtsregler) vorgesehen. Dieser wandelt die relativ geringe, von dem mindestens einen photovoltaischen Konverter gelieferte Spannung in eine mit dem verwendeten Akku kompatible Eingangsspannung von in der Regel 2 V bis 6 V um. Da diese Aufwärtswandler aber ebenfalls eine Spannung von in der Regel mindestens 0,35 V bis 0,75 V benötigen, sollte der verbaute photovoltaische Konverter oder die verbauten photovoltaischen Konverter in Kombination mit der Vorrichtung zum Aufladen eine solche Spannung von mindestens 0,35 V bis mindestens 0,75 V liefern. Dem Fachmann ist bekannt, welcher Typ von photovoltaischem Konverter in welchen Dimensionen bei welchen Lichtbedingungen eine derartige Spannung liefern kann. Zudem ist es möglich, mehrere photovoltaische Konverter zu verwenden und diese in Reihe zu schalten, um eine höhere Spannung zu erzielen.

Als **Sterilverpackung** sind diverse Kunststoffe geeignet, welche vorzugsweise transparent sind, als mikrobielle Barriere wirken, d.h. das darin befindliche sterilisierte Medizinprodukt für einen Zeitraum von mindestens 3 Jahren steril halten und eine Sterilisation des Medizinproduktes durch die Sterilverpackung hindurch zulassen.

Ob sich ein Material für die Verwendung als Sterilverpackung für die vorliegende Erfindung in Bezug auf die Barriere für Keime eignet, lässt sich wie im Folgenden beschrieben testen. Eine Petrischale mit Nährmedium für Bakterien (z.B. in Form von Agarose) wird in einer Verpackung aus dem zu testenden Material versiegelt. Anschließend wird die Petrischale samt Verpackung sterilisiert, vorzugsweise nach dem Verfahren, das auch für die erfindungsgemäßen Medizinprodukte verwendet wird. Anschließend wird das sterilisierte Gut in einer Expositionskammer einer definierten Keimbelastung (Keim-Spezies, Keimzahl, Dauer, Temperatur) ausgesetzt. Dann wird das sterilisierte Gut für eine bestimmte Zeit bei geeigneten Temperaturen inkubiert und anschließend eine eventuelle Keimbelastung bestimmt.

Ob sich ein Material für die Verwendung als Sterilverpackung für die vorliegende Erfindung in Bezug auf das Sterilisationsverfahren eignet, lässt sich wie im Folgenden beschrieben testen. In einer Petrischale wird auf Nährmedium für Bakterien (z.B. in Form von Agarose) eine definierten Menge Bakterien ausgebracht. Anschließend wird diese Petrischale in einer Verpackung aus dem zu testenden Material versiegelt. Danach wird die Petrischale samt Verpackung sterilisiert. Hierbei wird das Verfahren angewandt, das auch für die erfindungsgemäßen Medizinprodukte verwendet wird. Dann wird das sterilisierte Gut für eine bestimmte Zeit bei geeigneten Temperaturen inkubiert und anschließend eine eventuelle Keimbelastung bestimmt. Genaueres zu möglichen Testverfahren und Anforderungen an Sterilverpackungen sind in der Norm ISO 11607-1:2006 verzeichnet.

Die Sterilisation der erfindungsgemäßen Medizinprodukte erfolgt vorzugsweise bei Temperaturen unterhalb von 60°C, weiter bevorzugt unterhalb von 50°C, weiter bevorzugt unterhalb von 40°C, und noch weiter bevorzugt unterhalb von 30°C, mit einem Gas wie beispielsweise Wasserstoffperoxid, Ethylenoxid, Ozon, Formaldehyd und/oder Peressigsäure. Daher muss die Sterilverpackung durchlässig für das Sterilisationsgas sein.

Die vorliegende Erfindung umfasst folglich ein in einer Sterilverpackung befindliches sterilisiertes Medizinprodukt, wobei die Sterilverpackung durchlässig für Wasserstoffperoxid, Ethylenoxid, Ozon, Formaldehyd und/oder Peressigsäure ist.

Besonders bevorzugt werden hierbei Sterilisationsverfahren mit Gas, bei denen das zu sterilisierende Medizinprodukt nach der Sterilisation im trockenen Zustand vorliegt (auch als Trockenantiseptik bezeichnet).

In einer bevorzugten Ausführungsform der vorliegenden Erfindung ist die Sterilverpackung durchlässig für Wasserstoffperoxid, Ethylenoxid, Ozon und/oder Formaldehyd, am meisten bevorzugt für Wasserstoffperoxid und/oder Ethylenoxid.

In einer bevorzugten Ausführungsform der vorliegenden Erfindung wird das zunächst unsterile Medizinprodukt in einer zur Sterilisation geeigneten Verpackung verpackt, so dass man ein nicht steriles Medizinprodukt in einer Sterilverpackung erhält. Im Anschluss hieran erfolgt die Sterilisation des Medizinproduktes innerhalb der Sterilverpackung, so dass man ein sterilisiertes Medizinprodukt innerhalb der Sterilverpackung erhält. Da die Sterilisation der Einheit aus Verpackung und Medizinprodukt durch die Verpackung hindurch erfolgt und zudem Ausführungsformen des erfindungsgemäßen Medizinproduktes mit interner Energieversorgungseinheit zumeist thermolabil sind, werden zur Sterilisation Niedertemperaturverfahren wie u.a. mit Gas (z.B. Ethylenoxid) oder durch Plasma (z.B. mit Wasserstoffperoxid) bevorzugt. Besonders bevorzugt wird hierbei die Plasmasterilisation mittels Wasserstoffperoxid (H₂O₂) unter Verwendung eines Temperaturbereiches von 25°C bis 60°C, weiter bevorzugt von 30°C bis 55°C oder von 35°C bis 50°C. Durch die bei der Plasmasterilisation verwendeten vergleichsweise niedrigen Temperaturen werden besonders thermolabile Produkte schonend sterilisiert. Daher eignet sich dieses Verfahren besonders für die Sterilisation der erfindungsgemäßen Medizinprodukte.

Ein weiterer wichtiger Aspekt der vorliegenden Erfindung betrifft die Transparenz bzw. die Durchlässigkeit (Transmission) der Sterilverpackung für elektromagnetische Strahlung in einem Bereich des Spektrums, der von dem mindestens einen photovoltaischen Konverter, insbesondere Solarzelle oder Photodiode, zur Erzeugung von elektrischem Strom verwendet wird bzw. verwendet werden kann. Nur durch eine ausreichende Durchlässigkeit des Materials der Sterilverpackung kann daher eine effiziente Aufladung der internen Energieversorgungseinheit des Medizinproduktes durch den mindestens einen photovoltaischen Konverter gewährleistet werden. Diese Durchlässigkeit der Sterilverpackung muss mindestens in dem den zumindest einen photovoltaischen Konverter bedeckenden Bereich zum Zeitpunkt der geplanten Aufladung gewährleistet sein.

Folglich ist das erfindungsgemäße Medizinprodukt weiterhin dadurch charakterisiert, dass die Sterilverpackung insgesamt oder zumindest in dem den mindestens einen photovoltaischen Konverter bedeckenden Bereich eine mittlere Durchlässigkeit für Strahlung der Wellenlänge von 300 nm bis 1100 nm von ≥ 50%, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % aufweist.

Folglich ist das erfindungsgemäße Medizinprodukt weiterhin dadurch charakterisiert, dass die Sterilverpackung insgesamt oder zumindest in dem die mindestens eine Solarzelle bedeckenden Bereich eine mittlere Durchlässigkeit für Strahlung der Wellenlänge von 300 nm bis 1100 nm von ≥ 50%, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % aufweist.

Folglich ist das erfindungsgemäße Medizinprodukt weiterhin dadurch charakterisiert, dass die Sterilverpackung insgesamt oder zumindest in dem die mindestens eine Photodiode bedeckenden Bereich eine mittlere Durchlässigkeit für Strahlung der Wellenlänge von 300 nm bis 1100 nm von ≥ 50%, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % aufweist.

In einer Ausführungsform, in der eine kristalline Siliziumzelle oder eine Kupfer-Indium-Diselenid-Zelle verwendet wird, ist bevorzugt, dass die Sterilverpackung zumindest in dem die mindestens eine Solarzelle bedeckenden Bereich eine mittlere Durchlässigkeit für Strahlung der Wellenlänge von 700 nm bis 1100 nm von ≥ 50%, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % aufweist.

In einer Ausführungsform, in der eine amorphe Siliziumzelle oder eine Kupfer-Gallium-Diselenid-Zelle verwendet wird, ist bevorzugt, dass die Sterilverpackung zumindest in dem die mindestens eine Solarzelle bedeckenden Bereich eine mittlere Durchlässigkeit für Strahlung der Wellenlänge von 450 nm bis 750 nm von ≥ 50%, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % aufweist.

In einer Ausführungsform, in der eine Gallium-Arsenid-Zelle, eine Cadmium-Tellurid-Zelle oder eine Kupfer-Indium-Disulfid-Zelle verwendet wird, ist bevorzugt, dass die Sterilverpackung zumindest in dem die mindestens eine Solarzelle bedeckenden Bereich eine mittlere Durchlässigkeit für Strahlung der Wellenlänge von 500 nm bis 900 nm von ≥ 50%, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % aufweist.

| Verwendetes Material für die Solarzelle | Bevorzugte mittlere Durchlässigkeit der Sterilverpackung im Bereich des photovoltaischen Konverters |
|---|---|
| Kristallines Silizium, CuInSe₂ (Chalkopyrit) | 700 - 1100 nm |
| amorphes Silizium, CuGaSe₂ (Chalkopyrit) | 450 - 750 nm |
| CuInS₂, CdTe, GaAs | 500 - 900 nm |
| | |

| Verwendetes Material für die Photodiode | |
|---|---|
| Silizium | 190 - 1100 nm |
| Germanium | 400 - 1700 nm |

"Mittlere Durchlässigkeit", wie hierin verwendet, bedeutet, dass die Transmission im angegebenen Wellenlängenbereich im Mittel einen bestimmten minimalen prozentualen Wert erreicht. Es bedeutet nicht zwingend, dass für jede Wellenlänge in diesem Bereich dieser prozentuale Wert erreicht werden muss.

Der Begriff "insgesamt", wie hierin verwendet, bedeutet im Falle einer Sterilverpackung, die nur aus einem Teil und/oder aus einem Material besteht, dass die gesamte Sterilverpackung und nicht nur ein Teil von ihr eine wie hierin bereits offenbarte Durchlässigkeit für Strahlung eines hierin offenbarten Wellenlängenbereiches aufweist. Die Sterilverpackung kann aber auch aus zwei Teilen aus unterschiedlichen Materialien mit folglich unterschiedlichen Eigenschaften bestehen. In diesem Fall bedeutet "insgesamt", dass der Teil der Sterilverpackung, der für die Lichtdurchlässigkeit verantwortlich ist, eine wie hierin bereits offenbarte Durchlässigkeit für Strahlung eines hierin offenbarten Wellenlängenbereiches aufweist.

Die vorliegende Erfindung betrifft daher auch ein in einer Sterilverpackung befindliches sterilisiertes Medizinprodukt mit einer internen Energieversorgungseinheit und einem auf, an oder in der Oberfläche des Medizinproduktes befindlichen photovoltaischen Konverter, wobei die interne Energieversorgungseinheit durch den photovoltaischen Konverter aufladbar ist und die Sterilverpackung zumindest in dem den photovoltaischen Konverter bedeckenden Bereich eine mittlere Durchlässigkeit für Strahlung der Wellenlänge von 300 nm bis 1100 nm von mindestens 50 %, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % besitzt.

Die vorliegende Erfindung betrifft daher auch ein in einer Sterilverpackung befindliches sterilisiertes Medizinprodukt mit einer internen Energieversorgungseinheit und einer auf, an oder in der Oberfläche des Medizinproduktes befindlichen Solarzelle, wobei die interne Energieversorgungseinheit durch die Solarzelle aufladbar ist und die Sterilverpackung zumindest in dem die Solarzelle bedeckenden Bereich eine mittlere Durchlässigkeit für Strahlung der Wellenlänge von 300 nm bis 1100 nm von mindestens 50 %, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % besitzt.

Die vorliegende Erfindung betrifft daher auch ein in einer Sterilverpackung befindliches sterilisiertes Medizinprodukt mit einer internen Energieversorgungseinheit und einer auf, an oder in der Oberfläche des Medizinproduktes befindlichen Photodiode, wobei die interne Energieversorgungseinheit durch die Photodiode aufladbar ist und die Sterilverpackung zumindest in dem die Photodiode bedeckenden Bereich eine mittlere Durchlässigkeit für Strahlung der Wellenlänge von 300 nm bis 1100 nm von mindestens 50 %, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % besitzt.

Als mögliche Ausführungsformen der erfindungsgemäßen Sterilverpackung kommen mehrere Varianten in Betracht. Einerseits ist denkbar, dass die gesamte Sterilverpackung aus ein und demselben Material bzw. ein und derselben Materialzusammensetzung besteht. Somit muss dieses Material bzw. diese Materialzusammensetzung die bereits beschriebenen Eigenschaften hinsichtlich Durchlässigkeit für Sterilisationsgase und Durchlässigkeit für elektromagnetische Strahlung vereinen. Andererseits ist es auch denkbar, dass die Sterilverpackung aus mehreren Teilen mit unterschiedlicher Materialzusammensetzung besteht wie z.B. einem Blister aus Material A und einem Blisterdeckel oder einer Blisterabdeckung aus Material B. Hierbei muss zumindest ein Teil der Verpackung für Sterilisationsgase durchlässig sein und zumindest ein Teil der Verpackung durchlässig für elektromagnetische Strahlung sein. D.h. Material A (also z.B. der Blister) muss zumindest entweder durchlässig für ein Sterilisationsgas oder für die elektromagnetische Strahlung sein und Material B (also z.B. der Blisterdeckel oder die Blisterabdeckung) muss die zweite Eigenschaft ausweisen, also zumindest entweder durchlässig für die elektromagnetische Strahlung oder für ein Sterilisationsgas sein. Es ist hierbei durchaus denkbar, dass Teile der Verpackung beide Eigenschaften vereinen, aber auch, dass Teile der Verpackung nur eine der beiden Eigenschaften besitzen. In einer bevorzugten Ausführungsform der vorliegenden Erfindung befindet sich das Medizinprodukt in einem Blister aus Polypropylen, der eine Durchlässigkeit für Strahlung der Wellenlänge von 300 nm bis 1100 nm von mindestens 50 % besitzt. Dieser Blister wird vor der Sterilisation durch eine Abdeckung (auch Blisterdeckel) verschlossen, der aus Tyvek^{®} (hochdichte, beschichtete Polyethylenfasern) besteht und somit zwar nicht lichtdurchlässig dafür aber durchlässig für das Sterilisationsgas Wasserstoffperoxid ist.

Beispiele für geeignete Materialien für den Blister, d.h. für die Komponente der Sterilverpackung, welche durchlässig für die eingesetzte Strahlung ist, sind unter anderem Polyethylen (PE), Polyethylen hoher Dichte (PE-HD oder HDPE), Polypropylen (PP), Polystyren (PS), Polycarbonat (PC), Polyethylenterephthalat (PET), glykol-modifiziertes Polyethylenterephthalat (PETG), Polyvinylchlorid (PVC), Polyetheretherketon (PEEK) sowie Thermoform-Kunststoffe. Als Material für die Abdeckung des Blisters (auch Blisterdeckel) ist insbesondere Tyvek^{®} geeignet, d.h. für die Komponente der Sterilverpackung, welche durchlässig für das Sterilisationsgas ist. Dem Fachmann sind weitere Kunststoffe bekannt, aus denen der Blister bzw. dessen Abdeckung bestehen kann.

Eine bevorzugte Ausführungsform des in einer Sterilverpackung befindlichen sterilisierten Medizinproduktes mit einer internen Energieversorgungseinheit und mindestens einem auf, an oder in der Oberfläche des Medizinproduktes befindlichen photovoltaischen Konverter ist dadurch charakterisiert, dass die zur Aufladung auf den photovoltaischen Konverter auftreffende Lichtleistung mindestens 150 mW/cm², vorzugsweise mindestens 180 mW/cm² und weiter bevorzugt mindestens 200 mW/cm² betragen muss, um eine Aufladung der internen Energieversorgungseinheit zu gewährleisten. Dies ist auf die nachgeschaltete Elektronik des Medizinproduktes zurückzuführen.

Eine bevorzugte Ausführungsform des in einer Sterilverpackung befindlichen sterilisierten Medizinproduktes mit einer internen Energieversorgungseinheit und mindestens einer auf, an oder in der Oberfläche des Medizinproduktes befindlichen Solarzelle ist dadurch charakterisiert, dass die zur Aufladung auf die Solarzelle auftreffende **Lichtleistung** mindestens 150 mW/cm², vorzugsweise mindestens 180 mW/cm² und weiter bevorzugt mindestens 200 mW/cm² betragen muss, um eine Aufladung der internen Energieversorgungseinheit zu gewährleisten. Dies ist auf die nachgeschaltete Elektronik des Medizinproduktes zurückzuführen.

Eine bevorzugte Ausführungsform des in einer Sterilverpackung befindlichen sterilisierten Medizinproduktes mit einer internen Energieversorgungseinheit und mindestens einer auf, an oder in der Oberfläche des Medizinproduktes befindlichen Photodiode ist dadurch charakterisiert, dass die zur Aufladung auf die Photodiode auftreffende **Lichtleistung** mindestens 150 mW/cm², vorzugsweise mindestens 180 mW/cm² und weiter bevorzugt mindestens 200 mW/cm² betragen muss, um eine Aufladung der internen Energieversorgungseinheit zu gewährleisten. Dies ist auf die nachgeschaltete Elektronik des Medizinproduktes zurückzuführen.

Folglich ist ein weiterer wichtiger Aspekt der vorliegenden Erfindung, dass die Sterilverpackung insgesamt oder zumindest in dem den mindestens einen photovoltaischen Konverter bedeckenden Bereich derart durchlässig für Strahlung der Wellenlänge von 300 nm bis 1100 nm ist, dass die auf den mindestens einen photovoltaischen Konverter aufgestrahlte Lichtleistung mindestens 150 mW/cm², bevorzugt mindestens 170 mW/cm², weiter bevorzugt mindestens 190 mW/cm², noch weiter bevorzugt mindestens 200 mW/cm² beträgt.

Folglich ist ein weiterer wichtiger Aspekt der vorliegenden Erfindung, dass die Sterilverpackung insgesamt oder zumindest in dem die mindestens eine Solarzelle bedeckenden Bereich derart durchlässig für Strahlung der Wellenlänge von 300 nm bis 1100 nm ist, dass die auf die mindestens eine Solarzelle aufgestrahlte Lichtleistung mindestens 150 mW/cm², bevorzugt mindestens 170 mW/cm², weiter bevorzugt mindestens 190 mW/cm², noch weiter bevorzugt mindestens 200 mW/cm² beträgt.

Folglich ist ein weiterer wichtiger Aspekt der vorliegenden Erfindung, dass die Sterilverpackung insgesamt oder zumindest in dem die mindestens eine Photodiode bedeckenden Bereich derart durchlässig für Strahlung der Wellenlänge von 300 nm bis 1100 nm ist, dass die auf die mindestens eine Photodiode aufgestrahlte Lichtleistung mindestens 150 mW/cm², bevorzugt mindestens 170 mW/cm², weiter bevorzugt mindestens 190 mW/cm², noch weiter bevorzugt mindestens 200 mW/cm² beträgt.

Um eine ausreichende Durchlässigkeit der Sterilverpackung für elektromagnetische Strahlung in einem Bereich des Spektrums, der von dem mindestens einen photovoltaischen Konverter, insbesondere Solarzelle oder Photodiode, zur Erzeugung von elektrischem Strom verwendet werden kann, zu erreichen, ist ein weiterer wichtiger Aspekt die Wanddicke der Sterilverpackung, da mit steigender Wanddicke die Lichtverluste durch Absorption, Reflektion, Beugung und Brechung steigen. Daher sollen durch eine möglichst geringe Wanddicke der Sterilverpackung die Lichtverluste beim Durchtritt durch die Sterilverpackung minimiert werden.

Folglich ist das erfindungsgemäße Medizinprodukt in einer weiteren Ausführungsform dadurch charakterisiert, dass die Sterilverpackung eine Wanddicke im Bereich von 0,1 mm bis 10 mm, weiterhin bevorzugt von 0,3 mm bis 5 mm, am meisten bevorzugt von 0,5 mm bis 1 mm aufweist.

Ob sich ein Material für die Verwendung als Sterilverpackung für die vorliegende Erfindung in Bezug auf die Durchlässigkeit (Transmission) eignet lässt sich durch Messungen mit einen Spektrophotometer überprüfen. Das zu testende Material wird hierbei in der entsprechenden Dicke zwischen Lichtquelle und Detektor angebracht und unter definierten Bedingungen (z.B. Wellenlängenbereich, Lichtintensität) beleuchtet. Indem die vom Material durchgelassene und am Detektor ankommende Strahlungsintensität in Bezug zu einer Kontrollmessung ohne Materialprobe gesetzt wird, lässt sich die Durchlässigkeit (Transmission) bestimmen.

Eine wiederholte Exposition von Materialien, insbesondere von Kunststoffen, gegenüber Sonnenlicht, insbesondere von ultravioletter Strahlung (UV-Strahlung, ca. 10 nm - 380 nm) dazu führen kann, dass diese Materialien Schaden nehmen. Im Fall von Kunststoffen kann sich dies darin äußern, dass der Kunststoff spröde wird und/oder vergilbt. Eine derartige Veränderung des Verpackungsmaterials wäre natürlich nicht nur nachteilig für die Sterilität, sondern auch für die notwendige Durchlässigkeit für Strahlung, insbesondere von Strahlung im Bereich von 300 nm - 1100 nm.

Daher ist eine bevorzugte Ausführungsform der vorliegenden Erfindung dadurch charakterisiert, dass die Sterilverpackung eine hohe Beständigkeit für UV-Strahlung aufweist.

Eine weitere bevorzugte Ausführungsform der vorliegenden Erfindung ist dadurch charakterisiert, dass das in einer Sterilverpackung befindliche sterilisierte Medizinprodukt vollständig von einer weiteren, vorzugsweise unsterilen, Verpackung (Umverpackung) umgeben ist, welche undurchlässig für Strahlung, insbesondere von Strahlung im Bereich von 300 nm - 1100 nm ist.

Zusätzlich zu dem Schutz vor einer unnötigen und unter Umständen schädlichen Lichteinwirkung während der Lagerung des erfindungsgemäßen Produktes, können durch eine lichtundurchlässige Abdeckung des photovoltaischen Konverters, insbesondere Solarzelle oder Photodiode, auch ungewollte Kurzladephasen der internen Energieversorgungseinheit des Medizinproduktes verhindert werden, welche die Lebensdauer der internen Energieversorgungseinheit verringen können.

Eine weitere Ausführungsform der vorliegenden Erfindung betrifft das in einer Sterilverpackung befindliche sterilisierte Medizinprodukt, wobei die Sterilverpackung nur in dem den mindestens einen photovoltaischen Konverter bedeckenden Bereich eine Durchlässigkeit für Strahlung der Wellenlänge von 300 nm bis 1100 nm von ≥ 55%, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % aufweist. Folglich ist die Sterilverpackung in dem Bereich, der nicht den photovoltaischen Konverter bedeckt, undurchlässig für Strahlung, insbesondere undurchlässig für Strahlung im Wellenlängenbereich von 300 nm bis 1100 nm.

Eine weitere Ausführungsform der vorliegenden Erfindung betrifft das in einer Sterilverpackung befindliche sterilisierte Medizinprodukt, wobei die Sterilverpackung nur in dem die mindestens eine Solarzelle bedeckenden Bereich eine Durchlässigkeit für Strahlung der Wellenlänge von 300 nm bis 1100 nm von ≥ 55%, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % aufweist. Folglich ist die Sterilverpackung in dem Bereich, der nicht die Solarzelle bedeckt, undurchlässig für Strahlung, insbesondere undurchlässig für Strahlung im Wellenlängenbereich von 300 nm bis 1100 nm.

Eine weitere Ausführungsform der vorliegenden Erfindung betrifft das in einer Sterilverpackung befindliche sterilisierte Medizinprodukt, wobei die Sterilverpackung nur in dem die mindestens eine Photodiode bedeckenden Bereich eine Durchlässigkeit für Strahlung der Wellenlänge von 300 nm bis 1100 nm von ≥ 55%, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % aufweist. Folglich ist die Sterilverpackung in dem Bereich, der nicht die Photodiode bedeckt, undurchlässig für Strahlung, insbesondere undurchlässig für Strahlung im Wellenlängenbereich von 300 nm bis 1100 nm.

Die Formulierung "in dem den photovoltaischen Konverter bedeckenden Bereich", wie hierin verwendet, bezeichnet die Fläche der Sterilverpackung, die sich direkt gegenüber der licht-empfindlichen und lichtzugewandten Fläche des verbauten photovoltaischen Konverters oder der photovoltaischen Konverter befindet. Hierbei kann hinsichtlich der Ausdehnung dieser Fläche der Sterilverpackung eine Abweichung im Vergleich zur Ausdehnung der licht-empfindlichen Fläche des verbauten photovoltaischen Konverters / der photovoltaischen Konverter von bis zu ± 2 mm in Längs- als auch in Querrichtung toleriert werden.

Die Formulierung "in dem die Solarzelle bedeckenden Bereich", wie hierin verwendet, bezeichnet die Fläche der Sterilverpackung, die sich direkt gegenüber der licht-empfindlichen und lichtzugewandten Fläche der verbauten Solarzelle oder Solarzellen befindet. Hierbei kann hinsichtlich der Ausdehnung dieser Fläche der Sterilverpackung eine Abweichung im Vergleich zur Ausdehnung der licht-empfindlichen Fläche der verbauten Solarzelle / Solarzellen von bis zu ± 2 mm in Längs- als auch in Querrichtung toleriert werden.

Die Formulierung "in dem die Photodiode bedeckenden Bereich", wie hierin verwendet, bezeichnet die Fläche der Sterilverpackung, die sich direkt gegenüber der licht-empfindlichen und lichtzugewandten Fläche der verbauten Photodiode oder Photodioden befindet. Hierbei kann hinsichtlich der Ausdehnung dieser Fläche der Sterilverpackung eine Abweichung im Vergleich zur Ausdehnung der licht-empfindlichen Fläche der verbauten Photodiode / Photodioden von bis zu ± 2 mm in Längs- als auch in Querrichtung toleriert werden.

"Undurchlässig" und "nicht lichtdurchlässig", wie hierin verwendet, bedeutet, dass die mittlere Durchlässigkeit der Sterilverpackung für Strahlung der Wellenlänge von 300 nm bis 1100 nm unter 10 % liegt. Diese Definition ist anwendbar, wenn der Begriff "undurchlässig" und "nicht lichtdurchlässig" ohne einen konkreten Wellenlängenbereich genannt wird.

"Undurchlässig" und "nicht lichtdurchlässig", wie hierin verwendet, bedeutet, dass die Transmission der Sterilverpackung für den jeweils angegebenen Wellenlängenbereich so gering ist, dass die residuale durchtretende Strahlungsenergie nicht für die Stromerzeugung mittels photovoltaischem Konverter (insbesondere Solarzelle oder Photodiode) ausreichend ist. Diese Definition ist anwendbar, wenn der Begriff "undurchlässig" und "nicht lichtdurchlässig" mit einem konkreten Wellenlängenbereich genannt wird.

"Undurchlässig" und "nicht lichtdurchlässig", wie hierin verwendet, bedeutet, dass die Transmission der Sterilverpackung für den jeweils angegebenen Wellenlängenbereich so gering ist, dass die interne Energieversorgungseinheit nicht durch die residuale durchtretende Strahlungsenergie mittels des photovoltaischen Konverters / den photovoltaischen Konvertern (insbesondere Solarzelle oder Photodiode) aufladbar ist. Diese Definition ist anwendbar, wenn der Begriff "undurchlässig" und "nicht lichtdurchlässig" mit einem konkreten Wellenlängenbereich genannt wird.

Eine weitere Ausführungsform betrifft das erfindungsgemäße Medizinprodukt, wobei die Sterilverpackung zumindest in dem den mindestens einen photovoltaischen Konverter (insbesondere Solarzelle oder Photodiode) bedeckenden Bereich durch eine entfernbare und nicht lichtdurchlässige Abdeckung versehen ist. Diese Abdeckung kann auch so ausgestaltet sein, dass sie abnehmbar ist, um den Aufladevorgang mittels externer Lichtquelle durchzuführen und danach wieder angebracht werden kann, um während des Transportes und nachfolgender Lagerung vor unbeabsichtigter Bestrahlung durch Raumlicht oder Sonnenlicht zu schützen. Eine solche Abdeckung kann z.B. eine selbstklebende Folie sein.

Durch die Anbringung einer entfernbaren und nicht lichtdurchlässigen Abdeckung über dem photovoltaischen Konverter, insbesondere Solarzelle oder Photodiode, sollen in erster Linie bei wiederholten und nicht der gezielten Aufladung dienenden Expositionen mit Licht (z.B. durch Öffnen des Kühlschrankes, in dem das erfindungsgemäße Medizinprodukt gelagert wird) auftretende Kurzladephasen vermieden werden, die der Lebensdauer der internen Energieversorgungseinheit schaden können.

Da die Verpackung des Medizinproduktes derart erfolgt, dass der zumindest eine photovoltaische Konverter, insbesondere Solarzelle oder Photodiode, direkt unterhalb der Sterilverpackung positioniert ist, welche zudem den Durchtritt von Strahlung zur Erzeugung von elektrischem Strom mittels des mindestens einen photovoltaischen Konverters zulässt, kann nun durch eine externe Lichtquelle die interne Energieversorgungseinheit des Medizinproduktes aufgeladen werden. Dies geschient hierbei ohne die Sterilität der Sterilverpackung bzw. des Medizinproduktes zu beeinflussen. Die Aufladung kann hierbei entweder direkt vor dem Versand durch den Hersteller oder bevorzugt direkt vor der Verwendung durch den Anwender erfolgen. Zur Aufladung der internen Energieversorgungseinheit wird hierbei ein **Ladegerät** mit einer eingebauten Lichtquelle verwendet, welches ebenfalls Gegenstand der vorliegenden Erfindung ist und so konstruiert ist, dass es das in der Sterilverpackung befindliche Medizinprodukt formschlüssig aufnehmen kann und so eine starke räumliche Nähe zwischen der Lichtquelle des Ladegerätes und dem photovoltaischen Konverter des Medizinproduktes hergestellt wird. Hierdurch wird der Ladevorgang optimiert.

Ein weiterer Aspekt der vorliegenden Erfindung betrifft daher eine Vorrichtung zum Aufladen der internen Energieversorgungseinheit eines Medizinproduktes, wobei die Vorrichtung eine Wannenform oder Beckenform aufweist und an zumindest einer Innenfläche der Wanne oder des Beckens mindestens eine Lichtquelle zur Erzeugung von durch den mindestens einen photovoltaischen Konverter des Medizinproduktes in elektrischen Strom umwandelbarer Strahlung angeordnet ist und das in der Sterilverpackung befindliche sterilisierte Medizinprodukt formschlüssig in die Wanne oder in das Becken so aufnehmbar ist, dass die mindestens eine Lichtquelle und der mindestens eine photovoltaische Konverter sich gegenüber liegen.

Ein weiterer Aspekt der vorliegenden Erfindung betrifft daher eine Vorrichtung zum Aufladen der internen Energieversorgungseinheit eines Medizinproduktes, wobei die Vorrichtung eine Wannenform oder Beckenform aufweist und an zumindest einer Innenfläche der Wanne oder des Beckens mindestens eine Lichtquelle zur Erzeugung von durch die mindestens eine Solarzelle des Medizinproduktes in elektrischen Strom umwandelbarer Strahlung angeordnet ist und das in der Sterilverpackung befindliche sterilisierte Medizinprodukt formschlüssig in die Wanne oder in das Becken so aufnehmbar ist, dass die mindestens eine Lichtquelle und die mindestens eine Solarzelle sich gegenüber liegen.

Ein weiterer Aspekt der vorliegenden Erfindung betrifft daher eine Vorrichtung zum Aufladen der internen Energieversorgungseinheit eines Medizinproduktes, wobei die Vorrichtung eine Wannenform oder Beckenform aufweist und an zumindest einer Innenfläche der Wanne oder des Beckens mindestens eine Lichtquelle zur Erzeugung von durch die mindestens eine Photodiode des Medizinproduktes in elektrischen Strom umwandelbarer Strahlung angeordnet ist und das in der Sterilverpackung befindliche sterilisierte Medizinprodukt formschlüssig in die Wanne oder in das Becken so aufnehmbar ist, dass die mindestens eine Lichtquelle und die mindestens eine Photodiode sich gegenüber liegen.

Der Begriff **"Vorrichtung zum Aufladen der internen Energieversorgungseinheit",** wie hierin verwendet, ist gleichbedeutend mit dem stellenweise verwendeten Begriff "Ladegerät" zu sehen.

Eine bevorzugte Ausführungsform des in einer Sterilverpackung befindlichen sterilisierten Medizinproduktes mit einer internen Energieversorgungseinheit und mindestens einem auf, an oder in der Oberfläche des Medizinproduktes befindlichen photovoltaischen Konverter, insbesondere Solarzelle oder Photodiode, ist dadurch charakterisiert, dass die zur Aufladung auf den photovoltaischen Konverter auftreffende Lichtleistung mindestens 150 mW/cm² bis 200 mW/cm² betragen muss, um eine Aufladung der internen Energieversorgungseinheit zu gewährleisten. Dies ist auf die nachgeschaltete Elektronik des Medizinproduktes zurückzuführen. Die mittlere Strahlungsleistung der Sonne beträgt lediglich etwa 137 mW/cm². Unter Berücksichtigung der hinzukommenden Strahlungsverluste, die u.a. durch die nicht hundertprozentige Durchlässigkeit der Sterilverpackung sowie durch Beugungs- und Reflektionsverluste an Phasenübergängen bedingt sind, reicht die Sonneneinstrahlung nicht aus, um die Aufladung der internen Energieversorgungseinheit des Medizinproduktes zu gewährleisten. Daher ist für diese bevorzugte Ausführungsform der Erfindung die Verwendung eines speziellen Ladegerätes notwendig.

Eine bevorzugte Ausführungsform der erfindungsgemäßen Vorrichtung zum Aufladen der internen Energieversorgungseinheit eines Medizinproduktes, wobei das in der Sterilverpackung befindliche sterilisierte Medizinprodukt derart formschlüssig in die Wanne oder in das Becken aufnehmbar ist, dass der Abstand zwischen der mindestens einen Lichtquelle und dem mindestens einen photovoltaischen Konverter nicht mehr als vorzugsweise 10 mm, bevorzugt nicht mehr als 6 mm, weiter bevorzugt nicht mehr als 5 mm, noch weiter bevorzugt nicht mehr als 4 mm und insbesondere bevorzugt nicht mehr als 3 mm beträgt.

Eine weitere bevorzugte Ausführungsform der erfindungsgemäßen Vorrichtung zum Aufladen der internen Energieversorgungseinheit eines Medizinproduktes, wobei das in der Sterilverpackung befindliche sterilisierte Medizinprodukt derart formschlüssig in die Wanne oder in das Becken aufnehmbar ist, dass der Abstand zwischen der mindestens einen Lichtquelle und dem mindestens einen photovoltaischen Konverter zwischen 0,1 mm - 15 mm, weiter bevorzugt zwischen 0,5 mm - 10 mm, noch weiter bevorzugt zwischen 1,0 mm - 5,0 mm, am meisten bevorzugt zwischen 2,0 mm - 4,0 mm beträgt.

Eine weitere bevorzugte Ausführungsform der erfindungsgemäßen Vorrichtung zum Aufladen der internen Energieversorgungseinheit eines Medizinproduktes, wobei das in der Sterilverpackung befindliche sterilisierte Medizinprodukt derart formschlüssig in die Wanne oder in das Becken aufnehmbar ist, dass der mindestens eine photovoltaische Konverter von der mindestens einen Lichtquelle mit einer Lichtstärke von mindestens 150 mW/cm², bevorzugt von mindestens 170 mW/cm², weiter bevorzugt von mindestens 190 mW/cm², noch weiter bevorzugt von mindestens 200 mW/cm² bestrahlt wird.

Eine bevorzugte Ausführungsform der erfindungsgemäßen Vorrichtung zum Aufladen der internen Energieversorgungseinheit eines Medizinproduktes, wobei das in der Sterilverpackung befindliche sterilisierte Medizinprodukt derart formschlüssig in die Wanne oder in das Becken aufnehmbar ist, dass der Abstand zwischen der mindestens einen Lichtquelle und der mindestens einen Solarzelle nicht mehr als vorzugsweise 10 mm, bevorzugt nicht mehr als 6 mm, weiter bevorzugt nicht mehr als 5 mm, noch weiter bevorzugt nicht mehr als 4 mm und insbesondere bevorzugt nicht mehr als 3 mm beträgt.

Eine weitere bevorzugte Ausführungsform der erfindungsgemäßen Vorrichtung zum Aufladen der internen Energieversorgungseinheit eines Medizinproduktes, wobei das in der Sterilverpackung befindliche sterilisierte Medizinprodukt derart formschlüssig in die Wanne oder in das Becken aufnehmbar ist, dass der Abstand zwischen der mindestens einen Lichtquelle und der mindestens einen Solarzelle zwischen 0,1 mm - 15 mm, weiter bevorzugt zwischen 0,5 mm - 10 mm, noch weiter bevorzugt zwischen 1,0 mm - 5,0 mm, am meisten bevorzugt zwischen 2,0 mm - 4,0 mm beträgt.

Eine weitere bevorzugte Ausführungsform der erfindungsgemäßen Vorrichtung zum Aufladen der internen Energieversorgungseinheit eines Medizinproduktes, wobei das in der Sterilverpackung befindliche sterilisierte Medizinprodukt derart formschlüssig in die Wanne oder in das Becken aufnehmbar ist, dass die mindestens eine Solarzelle von der mindestens einen Lichtquelle mit einer Lichtstärke von mindestens 150 mW/cm², bevorzugt von mindestens 170 mW/cm², weiter bevorzugt von mindestens 190 mW/cm², noch weiter bevorzugt von mindestens 200 mW/cm² bestrahlt wird.

Eine bevorzugte Ausführungsform der erfindungsgemäßen Vorrichtung zum Aufladen der internen Energieversorgungseinheit eines Medizinproduktes, wobei das in der Sterilverpackung befindliche sterilisierte Medizinprodukt derart formschlüssig in die Wanne oder in das Becken aufnehmbar ist, dass der Abstand zwischen der mindestens einen Lichtquelle und der mindestens einen Photodiode nicht mehr als vorzugsweise 10 mm, bevorzugt nicht mehr als 6 mm, weiter bevorzugt nicht mehr als 5 mm, noch weiter bevorzugt nicht mehr als 4 mm und insbesondere bevorzugt nicht mehr als 3 mm beträgt.

Eine weitere bevorzugte Ausführungsform der erfindungsgemäßen Vorrichtung zum Aufladen der internen Energieversorgungseinheit eines Medizinproduktes, wobei das in der Sterilverpackung befindliche sterilisierte Medizinprodukt derart formschlüssig in die Wanne oder in das Becken aufnehmbar ist, dass der Abstand zwischen der mindestens einen Lichtquelle und der mindestens einen Photodiode zwischen 0,1 mm - 15 mm, weiter bevorzugt zwischen 0,5 mm - 10 mm, noch weiter bevorzugt zwischen 1,0 mm - 5,0 mm, am meisten bevorzugt zwischen 2,0 mm - 4,0 mm beträgt.

Eine weitere bevorzugte Ausführungsform der erfindungsgemäßen Vorrichtung zum Aufladen der internen Energieversorgungseinheit eines Medizinproduktes, wobei das in der Sterilverpackung befindliche sterilisierte Medizinprodukt derart formschlüssig in die Wanne oder in das Becken aufnehmbar ist, dass die mindestens eine Photodiode von der mindestens einen Lichtquelle mit einer Lichtstärke von mindestens 150 mW/cm², bevorzugt von mindestens 170 mW/cm², weiter bevorzugt von mindestens 190 mW/cm², noch weiter bevorzugt von mindestens 200 mW/cm² bestrahlt wird.

Der hierin verwendete Begriff **"Wannenform oder Beckenform"** bezeichnet ein dreidimensionales Objekt mit rechteckiger Grundfläche, bei dem eine der sechs Seiten des Objektes offen ist. Hierdurch entsteht ein Innenraum des Objektes mit fünf Innenflächen. Die Innenfläche, die parallel zur offenen Seite des Objektes liegt, wird als "Boden" bezeichnet. Die Ausgestaltung als rechteckiges Objekt ist hierbei nicht als limitierend anzusehen, sondern dient lediglich zur Verdeutlichung des Bauprinzips. Dem Fachmann ist bekannt, dass an Stelle einer rechteckigen Grundfläche auch weitere geometrische Formen möglich sind wie z.B. Dreieck, Quadrat, Vieleck, Kreis, Ellipse usw.. Zudem ist es möglich, die Kanten des Objektes in abgerundeter Form zu gestalten.

Der Begriff **"formschlüssig",** wie hierin verwendet, bedeutet, dass die Sterilverpackung oder zumindest der Bereich der Sterilverpackung, in dem sich der mindestens eine photovoltaische Konverter, insbesondere Solarzelle oder Photodiode, befindet, in der Form dem Innenraum der Wannenform oder Beckenform der Vorrichtung zum Aufladen des Medizinproduktes entspricht, jedoch in den Maßen geringfügig kleiner ist, so dass die Sterilverpackung oder zumindest der Teil der Sterilverpackung, in dem sich der mindestens eine photovoltaische Konverter befindet, passgenau vor allem in Bezug auf die Innenmaße an der Oberkante der offenen Seite in die Vorrichtung zum Aufladen des Medizinproduktes eingesetzt werden kann.

Der Begriff **"gegenüber liegen",** wie hierin verwendet, bedeutet, dass die licht-emittierende Fläche der zumindest einen Lichtquelle und die licht-sensitive Fläche des zumindest einen photovoltaischen Konverters, insbesondere Solarzelle oder Photodiode, direkt gegenüber liegen und einander zugewandt sind.

Der Begriff **"aufnehmbar",** wie hierin verwendet, bezeichnet wie bereits oben erwähnt, dass das erfindungsgemäße in einer Sterilverpackung befindliche sterilisierte Medizinprodukt in eine entsprechende in der Vorrichtung zum Aufladen befindliche Aussparung eingesetzt werden kann.

Bei der zumindest einen Lichtquelle kann es sich unabhängig voneinander um Lichtquellen handeln, die Gruppe umfassend: Glühlampen, Halogenglühlampen, Glimmlampen, Leuchtstofflampen, Kaltkathodenröhren, Laser, sowie Licht-emittierende Dioden (LEDs). Dem Fachmann sind weitere Lichtquellen bekannt, die für die Anwendung geeignet sind. In einer bevorzugten Ausführungsform handelt es bei der zumindest einen Lichtquelle um eine LED, weiterhin bevorzugt um eine LED mit einem CRI-Wert *(Colour Rendering Index,* auch Farbwiedergabeindex oder Rₐ-Wert) von ≥ 80, weiter bevorzugt mit einem CRI ≥ 90, noch weiter bevorzugt mit einem CRI ≥ 95. Weiterhin wird eine LED bevorzugt mit einer Farbtemperatur ≥ 2800 K, noch weiter bevorzugt wird eine LED mit einer Farbtemperatur ≥ 3000 K; am meisten bevorzugt wird eine LED mit einer Farbtemperatur ≥ 3200 K.

In einer weiteren bevorzugten Ausführungsform handelt es bei der zumindest einen Lichtquelle um eine LED, welche dadurch charakterisiert ist, dass sie ein Emissionsmaximum in dem Wellenlängenbereich aufweist, indem der verwendete photovoltaische Konverter ein Absorptionsmaximum aufweist.

In einer weiteren bevorzugten Ausführungsform handelt es bei der zumindest einen Lichtquelle um eine LED, welche dadurch charakterisiert ist, dass sie ein Emissionsmaximum in dem Wellenlängenbereich aufweist, indem die verwendete Solarzelle ein Absorptionsmaximum aufweist.

In einer weiteren bevorzugten Ausführungsform handelt es bei der zumindest einen Lichtquelle um eine LED, welche dadurch charakterisiert ist, dass sie ein Emissionsmaximum in dem Wellenlängenbereich aufweist, indem die verwendete Photodiode ein Absorptionsmaximum aufweist.

Durch die Verwendung einer LED als Lichtquelle wird das Auftreten von längerwelliger Infrarotstrahlung (d.h. Wellenlänge > 1400 nm) und somit eine starke Erwärmung des Ladegerätes und des Medizinproduktes minimiert. Bei der Aufladung des Medizinproduktes soll eine Temperatur sowohl des Medizinproduktes als auch der Sterilverpackung von unter 30 °C beibehalten werden. In weiteren Ausführungen der vorliegenden Erfindung ist eine Kühlungsvorrichtung für das erfindungsgemäße Ladegerät bzw. die erfindungsgemäße Lichtquelle des Ladegerätes vorgesehen. Diese Kühlungsvorrichtung kann in bevorzugten Ausführungsformen der vorliegenden Erfindung u.a. durch eine Wasserkühlung, eine Luftkühlung (z.B. über einen Ventilator) oder durch wärme-ableitende Metallelemente realisiert sein. Dem Fachmann sind weitere Methoden zur Kühlung elektronischer Geräte bekannt, die in der vorliegenden Erfindung Verwendung finden können.

In einer bevorzugten Ausführungsform der erfindungsgemäßen Vorrichtung zum Aufladen der internen Energieversorgungseinheit eines Medizinproduktes ist auf der nach außen weisenden, licht-emittierenden Fläche der Lichtquelle eine Linse angebracht. Es ist weiterhin bevorzugt, wenn die verwendete Linse eine Dicke von 0,1 mm bis 10 mm, noch bevorzugter von 0,3 mm bis 5 mm, am meisten bevorzugt von 0,5 mm bis 3 mm aufweist. Durch eine möglichst geringe Linsendicke sollen hierbei die Lichtverluste beim Durchtritt durch die Linse minimiert werden. Sofern die licht-emittierende Fläche der Lichtquelle größer als die lichtsensitive Fläche des photovoltaischen Konverters ist, ist die bevorzugte Ausführungsform der Linse eine Sammellinse. Diese dient dazu, das emittierte Licht auf die geringere Fläche des photovoltaischen Konverters zu bündeln und so die Stromerzeugung zu optimieren. Sofern die licht-emittierende Fläche der Lichtquelle kleiner als die lichtsensitive Fläche des photovoltaischen Konverters ist, ist die bevorzugte Ausführungsform der Linse eine Zerstreuungslinse. Diese dient dazu, das emittierte Licht auf die größere Fläche des photovoltaischen Konverters zu verteilen und so die Stromerzeugung zu optimieren. Sofern die licht-emittierende Fläche der Lichtquelle größer als die lichtsensitive Fläche der Solarzelle ist, ist die bevorzugte Ausführungsform der Linse eine Sammellinse. Diese dient dazu, das emittierte Licht auf die geringere Fläche der Solarzelle zu bündeln und so die Stromerzeugung zu optimieren. Sofern die licht-emittierende Fläche der Lichtquelle kleiner als die lichtsensitive Fläche der Solarzelle ist, ist die bevorzugte Ausführungsform der Linse eine Zerstreuungslinse. Diese dient dazu, das emittierte Licht auf die größere Fläche der Solarzelle zu verteilen und so die Stromerzeugung zu optimieren.

Sofern die licht-emittierende Fläche der Lichtquelle größer als die lichtsensitive Fläche der Photodiode ist, ist die bevorzugte Ausführungsform der Linse eine Sammellinse. Diese dient dazu, das emittierte Licht auf die geringere Fläche der Photodiode zu bündeln und so die Stromerzeugung zu optimieren. Sofern die licht-emittierende Fläche der Lichtquelle kleiner als die lichtsensitive Fläche der Photodiode ist, ist die bevorzugte Ausführungsform der Linse eine Zerstreuungslinse. Diese dient dazu, das emittierte Licht auf die größere Fläche der Photodiode zu verteilen und so die Stromerzeugung zu optimieren.

Es sind Ausführungsformen der vorliegenden Erfindung vorstellbar, bei denen nicht nur ein sondern mehrere photovoltaische Konverter verwendet werden. Hierbei ist eine bevorzugte Ausführungsform, dass es sich bei den mehreren photovoltaischen Konvertern entweder um Solarzellen oder um Photodioden handelt. In einer weiteren bevorzugten Ausführungsform handelt es sich bei den mehreren photovoltaischen Konvertern um mindestens eine Solarzelle und mindestens eine Photodiode. Bei der Zusammensetzung aus mehreren photovoltaischen Konvertern sind natürlich auch ungleichmäßige Verhältnisse zwischen Solarzellen und Photodioden möglich.

In einer bevorzugten Ausführungsform der vorliegenden Erfindungen beinhaltet das erfindungemäße Medizinprodukt eine Solarzelle und die Vorrichtung zum Aufladen eine Lichtquelle, die so angeordnet sind, dass die eine Lichtquelle und die eine Solarzelle sich gegenüber liegen. Es sind aber natürlich auch Ausführungsformen denkbar, in denen mehrere Solarzellen und/oder mehrere Lichtquellen vorhanden sind. Sind mehrere Lichtquellen und nur eine Solarzelle vorhanden, so ist bevorzugt, dass die Lichtquellen so angeordnet sind, dass sie eine gemeinsame licht-emittierende Fläche bilden, die der Fläche der Solarzelle gegenüber liegt. Sind mehrere Solarzellen und nur eine Lichtquelle vorhanden, so ist bevorzugt, dass die Solarzellen so angeordnet sind, dass sie eine gemeinsame licht-sensitive Fläche bilden, die der Lichtquelle gegenüber liegt. Sind mehrere Lichtquellen und mehrere Solarzellen vorhanden, so ist bevorzugt, dass die Lichtquellen so angeordnet sind, dass sie eine gemeinsame licht-emittierende Fläche bilden und die Solarzellen so angeordnet sind, dass sie eine gemeinsame licht-sensitive Fläche bilden und sich die beiden Flächen gegenüber liegen. In einer weiteren, wenngleich weniger bevorzugten, Ausführungsform, in der mehrere Lichtquellen und mehrere Solarzellen vorhanden sind, sind sowohl die mehreren Solarzellen als auch die mehreren Lichtquellen auf unterschiedliche Bereiche des Medizinproduktes bzw. der Vorrichtung zum Aufladen verteilt, so dass aber immer zumindest eine Solarzelle mindestens einer Lichtquelle gegenüber liegt. Im Übrigen sind natürlich die oben beschriebenen Anordnungen im Falle unterschiedlicher Verhältnisse von photovoltaischen Konvertern und Lichtquellen nicht nur mit Solarzellen, sondern auch mit Photodioden und ebenso bei einer Verwendung von sowohl Solarzellen als auch Photodioden denkbar.

Des Weiteren beschreibt die vorliegende Erfindung Vorrichtungen, wie hierin offenbart, zum Aufladen der internen Energieversorgungseinheit eines Medizinproduktes mit formschlüssig aufgenommenen Medizinprodukten, wie hierin ebenfalls bereits offenbart.

Ein weiterer Aspekt der Erfindung betrifft ein **Verfahren** zum Aufladen der internen Energieversorgungseinheit eines Medizinproduktes, wobei der mindestens eine photovoltaische Konverter der Strahlung mindestens einer externen Lichtquelle ausgesetzt wird und die Strahlung der mindestens einen externen Lichtquelle die Sterilverpackung passiert, ohne die Sterilität zu beeinflussen und von dem mindestens einen photovoltaischen Konverter in elektrischen Strom umgewandelt wird, der zum Aufladen der internen Energieversorgungseinheit dient. Bei diesem Verfahren muss das Medizinprodukt zum Aufladen nicht aus der Sterilverpackung genommen werden, d.h. die Sterilität wird nicht zerstört und es erfolgt keine Kontamination des Medizinproduktes, wodurch eine erneute Sterilisation nach dem Aufladevorgang vermieden wird.

Eine bevorzugte Ausführungsform des erfindungsgemäßen Verfahrens zum Aufladen ist dadurch charakterisiert, dass der mindestens eine photovoltaischen Konverter von der mindestens einen Lichtquelle mit einer Lichtstärke von mindestens 150 mW/cm², bevorzugt von mindestens 170 mW/cm², weiter bevorzugt von mindestens 190 mW/cm², noch weiter bevorzugt von mindestens 200 mW/cm² bestrahlt wird.

Eine bevorzugte Ausführungsform des erfindungsgemäßen Verfahrens zum Aufladen ist dadurch charakterisiert, dass die Sterilverpackung insgesamt oder zumindest in dem den mindestens einen photovoltaischen Konverter bedeckenden Bereich eine mittlere Durchlässigkeit für Strahlung der Wellenlänge von 300 nm bis 1100 nm von ≥ 50 %, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % aufweist.

Ein weiterer Aspekt der vorliegenden Erfindung betrifft ein Verfahren zum Aufladen einer internen Energieversorgungseinheit eines in einer Sterilverpackung befindlichen Medizinproduktes mit mindestens einem auf, an oder in der Oberfläche des Medizinproduktes befindlichen photovoltaischen Konverter, wobei der mindestens eine photovoltaischen Konverter der Strahlung zumindest einer externen Lichtquelle ausgesetzt wird und die Strahlung der zumindest einen externen Lichtquelle die Sterilverpackung passiert ohne die Sterilität zu beeinflussen und von dem mindestens einen photovoltaischen Konverter in elektrischen Strom umgewandelt wird, der zum Aufladen der internen Energieversorgungseinheit dient.

Ein weiterer Aspekt der Erfindung betrifft ein Verfahren zum Aufladen der internen Energieversorgungseinheit eines Medizinproduktes, wobei die mindestens eine Solarzelle der Strahlung mindestens einer externen Lichtquelle ausgesetzt wird und die Strahlung der mindestens einen externen Lichtquelle die Sterilverpackung passiert, ohne die Sterilität zu beeinflussen und von der mindestens einen Solarzelle in elektrischen Strom umgewandelt wird, der zum Aufladen der internen Energieversorgungseinheit dient. Bei diesem Verfahren muss das Medizinprodukt zum Aufladen nicht aus der Sterilverpackung genommen werden, d.h. die Sterilität wird nicht zerstört und es erfolgt keine Kontamination des Medizinproduktes, wodurch eine erneute Sterilisation nach dem Aufladevorgang vermieden wird.

Eine bevorzugte Ausführungsform des erfindungsgemäßen Verfahrens zum Aufladen ist dadurch charakterisiert, dass die mindestens eine Solarzelle von der mindestens einen Lichtquelle mit einer Lichtstärke von mindestens 150 mW/cm², bevorzugt von mindestens 170 mW/cm², weiter bevorzugt von mindestens 190 mW/cm², noch weiter bevorzugt von mindestens 200 mW/cm² bestrahlt wird.

Eine bevorzugte Ausführungsform des erfindungsgemäßen Verfahrens zum Aufladen ist dadurch charakterisiert, dass die Sterilverpackung insgesamt oder zumindest in dem die mindestens eine Solarzelle bedeckenden Bereich eine mittlere Durchlässigkeit für Strahlung der Wellenlänge von 300 nm bis 1100 nm von ≥ 50 %, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % aufweist.

Ein weiterer Aspekt der vorliegenden Erfindung betrifft ein Verfahren zum Aufladen einer internen Energieversorgungseinheit eines in einer Sterilverpackung befindlichen Medizinproduktes mit mindestens einer auf, an oder in der Oberfläche des Medizinproduktes befindlichen Solarzelle, wobei die mindestens eine Solarzelle der Strahlung zumindest einer externen Lichtquelle ausgesetzt wird und die Strahlung der zumindest einen externen Lichtquelle die Sterilverpackung passiert ohne die Sterilität zu beeinflussen und von der mindestens einen Solarzelle in elektrischen Strom umgewandelt wird, der zum Aufladen der internen Energieversorgungseinheit dient.

Ein weiterer Aspekt der Erfindung betrifft ein Verfahren zum Aufladen der internen Energieversorgungseinheit eines Medizinproduktes, wobei die mindestens eine Photodiode der Strahlung mindestens einer externen Lichtquelle ausgesetzt wird und die Strahlung der mindestens einen externen Lichtquelle die Sterilverpackung passiert, ohne die Sterilität zu beeinflussen und von der mindestens einen Photodiode in elektrischen Strom umgewandelt wird, der zum Aufladen der internen Energieversorgungseinheit dient. Bei diesem Verfahren muss das Medizinprodukt zum Aufladen nicht aus der Sterilverpackung genommen werden, d.h. die Sterilität wird nicht zerstört und es erfolgt keine Kontamination des Medizinproduktes, wodurch eine erneute Sterilisation nach dem Aufladevorgang vermieden wird.

Eine bevorzugte Ausführungsform des erfindungsgemäßen Verfahrens zum Aufladen ist dadurch charakterisiert, dass die mindestens eine Photodiode von der mindestens einen Lichtquelle mit einer Lichtstärke von mindestens 150 mW/cm², bevorzugt von mindestens 170 mW/cm², weiter bevorzugt von mindestens 190 mW/cm², noch weiter bevorzugt von mindestens 200 mW/cm² bestrahlt wird.

Eine bevorzugte Ausführungsform des erfindungsgemäßen Verfahrens zum Aufladen ist dadurch charakterisiert, dass die Sterilverpackung insgesamt oder zumindest in dem die mindestens eine Photodiode bedeckenden Bereich eine mittlere Durchlässigkeit für Strahlung der Wellenlänge von 300 nm bis 1100 nm von ≥ 50 %, weiterhin bevorzugt von ≥60 %, noch weiter bevorzugt von ≥70 %, weiterhin bevorzugt von ≥75 %, noch weiter bevorzugt von ≥80 % und am meisten bevorzugt von ≥85 % aufweist.

Ein weiterer Aspekt der vorliegenden Erfindung betrifft ein Verfahren zum Aufladen einer internen Energieversorgungseinheit eines in einer Sterilverpackung befindlichen Medizinproduktes mit mindestens einer auf, an oder in der Oberfläche des Medizinproduktes befindlichen Photodiode, wobei die mindestens eine Photodiode der Strahlung zumindest einer externen Lichtquelle ausgesetzt wird und die Strahlung der zumindest einen externen Lichtquelle die Sterilverpackung passiert ohne die Sterilität zu beeinflussen und von der mindestens einen Photodiode in elektrischen Strom umgewandelt wird, der zum Aufladen der internen Energieversorgungseinheit dient.

Der wesentliche **Vorteil** der vorliegenden Erfindung besteht in der Möglichkeit, sterile Produkte mit einer internen Energieversorgungseinheit aufzuladen, ohne dass bei dem Aufladevorgang die Sterilität des Produktes beeinträchtigt wird. Dies ermöglicht es, besonders Medizinprodukte mit thermolabilen Akkus, deren Ladezustand durch lange Lagerung, Transport und erhöhte Temperaturen z.B. während des Sterilisationsprozesses vermindert wird, direkt vor der Verwendung vollständig aufzuladen, so dass zum benötigten Zeitpunkt die höchstmögliche Energie zur Verfügung steht.

### Figurenbeschreibung

- **Fig.1:**: zeigt die perspektivische Ansicht eines Ausführungsbeispiels des erfindungsgemäßen in einer Sterilverpackung befindlichen sterilisierten Medizinproduktes und des hiervon getrennten erfindungsgemäßen Ladegerätes.
- **Fig. 2:**: zeigt in einer perspektivischen Ansicht das Ausführungsbeispiel von Fig. 1, wobei jedoch das in einer Sterilverpackung befindliche sterilisierte Medizinprodukt in das Ladegerät eingesetzt ist.
- **Fig. 3:**: zeigt eine perspektivische Ansicht eines Querschnittes von Fig. 2.
- **Fig. 4:**: zeigt einen vergrößerten Ausschnitt aus Fig. 3 in Vorderansicht.
- **Fig. 5:**: zeigt einen vergrößerten Ausschnitt aus Fig. 4 in Vorderansicht.
- **Fig. 6:**: zeigt ähnlich wie Fig. 5 einen Querschnitt eines in einer Sterilverpackung befindlichen Medizinproduktes, welches in ein Ladegerät eingesetzt ist. Im Unterschied zu Fig. 5 zeigt Fig. 6 eine Ausführungsform, in der die Solarzelle (80) mit einer Steuerungseinheit (82) über elektrische Kontakte (81) abtrennbar mit dem Medizinprodukt (30) verbunden sind.

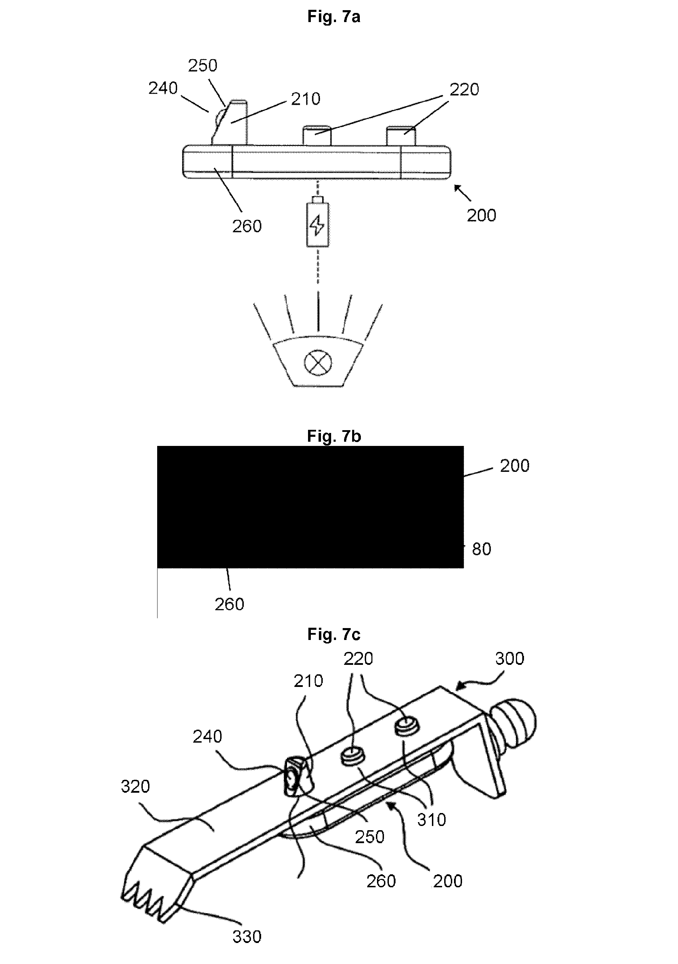

- **Fig. 7:**: zeigt eine bevorzugte Ausführungsform des erfindungsgemäßen Medizinproduktes in Form einer Beleuchtungsvorrichtung für chirurgische Instrumente (200) mit einem Zapfen (210) und zwei weiteren Befestigungsansätzen (220) in einer Seitenansicht (Fig. 7a). In einer Ansicht von unten (Fig. 7b) ist zu erkennen, dass eine Solarzelle (80) in die Unterseite des transparenten Gehäuses (260) der Beleuchtungsvorrichtung (200) integriert ist. Die Solarzelle dient zur Aufladung eines Akkumulators innerhalb des Gehäuses (260), der dann wiederum als Stromquelle für das Leuchtmittel im Kopf des Zapfens (210) dient. Fig. 7c zeigt die bevorzugte Ausführungsform als Beleuchtungsvorrichtung (200) eingesetzt in ein chirurgisches Instrument (300) in Gestalt eines Sperrerblattes, dessen Vorderseite (320) in perspektivischer Ansicht zu sehen ist.

### Beispiele

Im Folgenden wird die Erfindung anhand von Beispielen erläutert, die in den Figuren dargestellt sind.

Dies soll allein dem besseren Verständnis der Erfindung dienen, ohne dass die Erfindung auf die gezeigten Beispiele beschränkt wäre. Weitere Beispiele kann der Fachmann aufgrund seines allgemeinen Fachwissens mit Hilfe der vorausgegangenen Beschreibung auffinden.

### Beispiel 1:

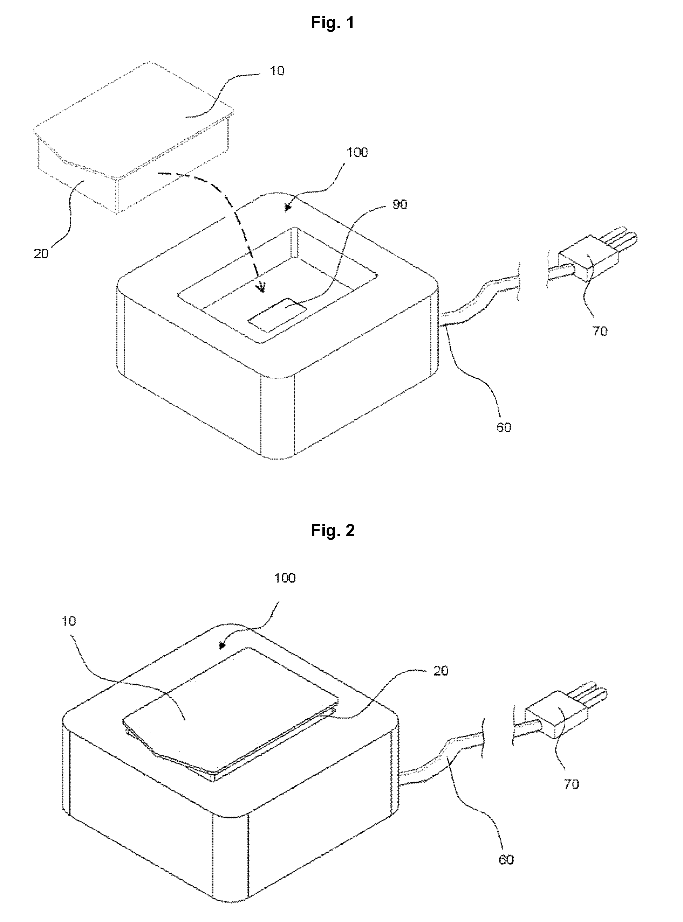

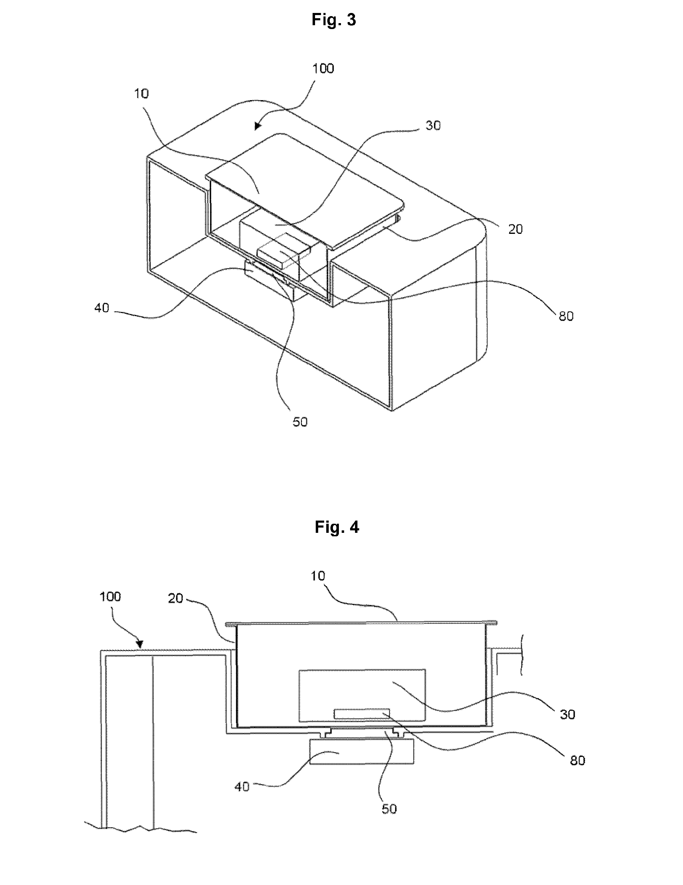

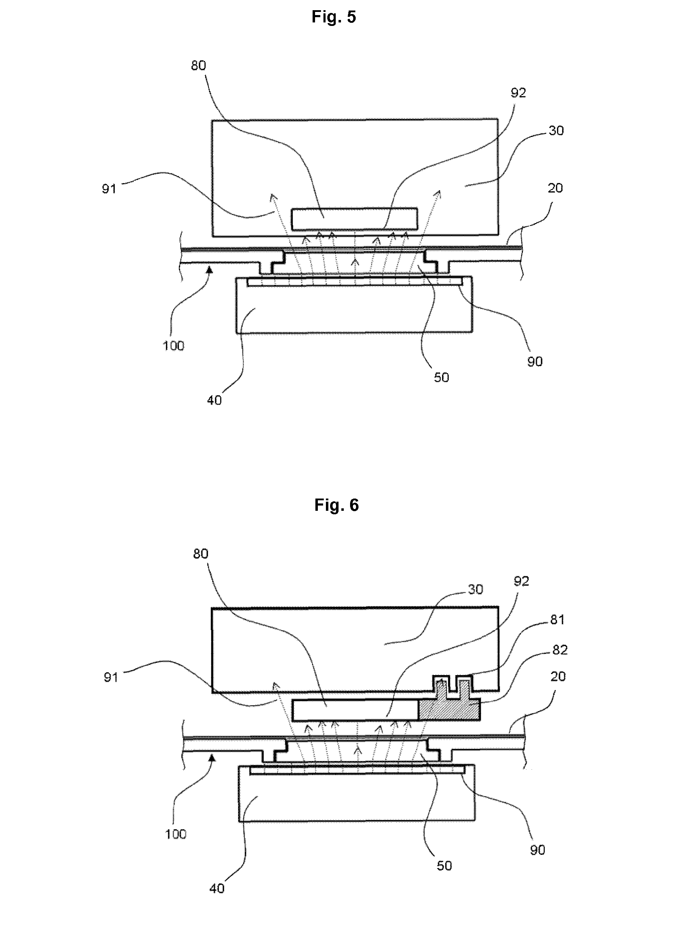

Beispiel 1 stellt eine exemplarische und stilisierte Ausführungsform des erfindungsgemäßen in einer Sterilverpackung befindlichen sterilisierten Medizinproduktes mit einer internen Energieversorgungseinheit und mindestens einem auf, an oder in der Oberfläche des Medizinproduktes befindlichen photovoltaischen Konverter sowie dem dazugehörigen erfindungsgemäßen Ladegerät dar. Fig. 1 zeigt in einer perspektivischen Außenansicht das Ladegerät (100) mit Netzkabel (60) und Netzstecker (70) sowie getrennt hiervon die Sterilverpackung in Form eines Blisters (20), der das Medizinprodukt enthält. In Fig. 2 ist ebenfalls in einer perspektivischen Außenansicht das Ladegerät (100) mit Netzkabel (60) und Netzstecker (70) zu erkennen, die Sterilverpackung in Form eines Blisters (20) wurde jedoch formschlüssig in das Ladegerät eingesetzt, so dass der Blister-Deckel (10) die Öffnung des Ladegerätes verdeckt. In Fig. 3 ist nun in perspektivischer Ansicht ein Querschnitt des Ladegerätes (100) samt eingesetzter Sterilverpackung (20) zu sehen. Fig. 4 zeigt einen vergrößerten Ausschnitt dieses Querschnitts in Vorderansicht. Man erkennt nun die am Boden der wannenförmigen Vertiefung des Ladegerätes (100) befindliche Lade-Lichtquelle (40) mit einer sich darüber befindlichen Linse (50). In starker räumlicher Nähe befindet sich über der Linse die eingesetzte Sterilverpackung (20) sowie das darin enthaltende Medizinprodukt (30), in das auf der der Lade-Lichtquelle zugewandten Seite ein photovoltaischer Konverter in Form einer Solarzelle (80) integriert ist. Fig. 5 zeigt eine Detailvergrößerung aus Fig. 4. Es ist zu erkennen, wie Licht (91), das von der Licht-emittierenden Fläche der Lade-Lichtquelle (90) ausgeht, von der darüber liegenden Linse (50) gebrochen wird, die Sterilverpackung (20) passiert und schließlich auf der lichtsensitiven lichtzugewandten Fläche der Solarzelle (92) auftrifft. Das Licht, das auf der lichtsensitiven lichtzugewandten Fläche der Solarzelle (92) auftrifft dient hierbei zur Aufladung der internen Energieversorgungseinheit des Medizinproduktes (30).

### Beispiel 2:

Ein Ausführungsbeispiel für ein Medizinprodukt mit einer internen Energieversorgungseinheit und mindestens einem auf, an der Oberfläche des Medizinproduktes befindlichen, abnehmbaren photovoltaischen Konverter ist in Fig. 6 dargestellt. Bei dem Medizinprodukt handelt es sich in diesem Fall um eine Vorrichtung zur Freisetzung eines Wirkstoffs. Der photovoltaische Konverter in Form einer Solarzelle (80) dient zur Aufladung eines Akkumulators innerhalb des Gehäuses des Medizinproduktes (30), der dann wiederum als Stromquelle für die integrierte Pumpvorrichtung dient. Die Solarzelle ist mit einer elektronischen Steuerungseinheit (82) verbunden, die sich ebenfalls außerhalb des Medizinprodukts befindet und zusammen mit der Solarzelle (80) als Solarmodul bezeichnet wird. Das Solarmodul ist über elektrische Kontakte (81) mit dem Medizinprodukt (30) elektrisch verbunden. Die Haltbarkeit dieser elektrischen Verbindung kann z.B. durch mechanischen oder magnetischen Kraftschluss unterstützt werden. Nach Trennen des Medizinprodukts (30) vom Solarmodul (80 + 82) können die elektrischen Kontakte (81) am Medizinprodukt versiegelt werden. Die Größe des Medizinprodukts zur Wirkstofffreisetzung kann durch Abtrennen des Solarmoduls deutlich verkleinert werden und ist dadurch besser für die Implantation geeignet.

### Beispiel 3:

Ein Ausführungsbeispiel für ein Medizinprodukt mit einer internen Energieversorgungseinheit und mindestens einem auf, an oder in der Oberfläche des Medizinproduktes befindlichen photovoltaischen Konverter ist in Fig. 7 dargestellt. Bei dem Medizinprodukt handelt es sich in diesem Fall um eine Beleuchtungsvorrichtung für chirurgische Instrumente (200). Wie in Fig. 7a in Seitenansicht dargestellt, besitzt die Beleuchtungsvorrichtung (200) ein kastenförmiges Gehäuse (260), das zwei zylindrisch ausgeprägte Befestigungsansätze (220) und einen zylindrisch und abgeschrägt ausgebildeten Zapfen (210) mit einer internen Lichtquelle, einer Lichtaustrittsöffnung (250) sowie einer lichtdurchlässigen Abdeckung (240) trägt. Das Gehäuse (260) ist hier gemeinsam mit den Befestigungsansätzen (220), dem Zapfen (210) und der lichtdurchlässigen Abdeckung (240) aus einem biokompatiblen Kunststoff hergestellt. Durch die zylindrische Form des Zapfens (210) sowie der Befestigungsansätze (220) klemmen diese optimal per Reibschluss sowohl in kreisförmigen Bohrungen als auch in Langlöchern, die bei dem in Fig. 7c dargestellten exemplarischen Instrument als Fensterungen ausgebildet sein können, oder in sonstig am Instrument vorhandenen Durchgangsöffnungen. Fig. 7b zeigt eine Unteransicht der Beleuchtungsvorrichtung (200) und offenbart einen photovoltaischen Konverter in Form einer Solarzelle (80), die auf der Unterseite in das transparente Gehäuse (260) der Beleuchtungsvorrichtung (200) integriert ist. Die Solarzelle (80) dient zur Aufladung eines Akkumulators innerhalb des Gehäuses (260), der dann wiederum als Stromquelle für das Leuchtmittel im Kopf des Zapfens (210) dient. Fig. 7c zeigt in perspektivischer Ansicht exemplarisch wie die Beleuchtungsvorrichtung (200) an einem Sperrerblatt (300) mit einem abgekantetem, gezinkten unteren Abschnitt (330) befestigt ist. Die Lichtaustrittsöffnung (250) weist eine optimale Position zur Ausleuchtung des Operationsfeldes auf, denn sie ist so nah wie möglich zum Operationsfeld angeordnet. Die Oberfläche der Beleuchtungsvorrichtung (200), auf der der Zapfen (210) sowie die Befestigungsansätze (220) angebracht sind, hat vollflächig Kontakt mit der Rückseite des Sperrerblatts (300), wobei die Befestigungsansätze (220) und der Zapfen (210) die Fensterung (310) des Sperrerblatts durchgreifen. Die Beleuchtungsvorrichtung (200) ist optimal an die längliche Form des Sperrerblatts (300) angepasst. Die Beleuchtungsvorrichtung (200) ist an der oberen Blatthälfte des Sperrerblatts (300) befestigt. Hierdurch ist zwischen den Zinken des Abschnitts (330) und der Beleuchtungsvorrichtung (200) genügend Raum für das mit dem Sperrerblatt (300) zurückgehaltene Gewebe. Die Wirkung der Zinken (330) wird durch die Anwesenheit des Gehäuses (260) nicht beeinträchtigt.

### Bezugszeichenliste

- **10**: Blister-Deckel
- **20**: Sterilverpackung (z. B. Blister)
- **30**: Medizinprodukt
- **40**: Lade-Lichtquelle
- **50**: Linse
- **60**: Netzkabel
- **70**: Netzstecker
- **80**: Solarzelle
- **81**: elektrische Kontakte
- **82**: Steuerungseinheit
- **90**: Licht-emittierende Fläche der Lade-Lichtquelle
- **91**: Licht
- **92**: Lichtsensitive lichtzugewandte Fläche der Solarzelle
- **100**: Ladegerät
- **200**: Beleuchtungsvorrichtung für chirurgische Instrumente
- **210**: Zapfen mit integrierter Lichtquelle
- **220**: Befestigungsansatz
- **240**: lichtdurchlässige Abdeckung / Linse
- **250**: Lichtaustrittsöffnung
- **260**: Gehäuse
- **300**: chirurgisches Instrument (hier: Sperrerblatt)
- **310**: Fensterung
- **320**: Vorderseite des Sperrerblattes
- **330**: gezinkter Abschnitt des Sperrerblattes

## Patentansprüche

1. In einer Sterilverpackung befindliches **sterilisiertes** Medizinprodukt mit einer internen Energieversorgungseinheit und mindestens einem auf, an oder in der Oberfläche des Medizinproduktes befindlichen photovoltaischen Konverter, wobei die interne Energieversorgungseinheit durch den mindestens einen photovoltaischen Konverter aufladbar ist und die Sterilverpackung zumindest in dem den mindestens einen photovoltaischen Konverter bedeckenden Bereich durchlässig für Strahlung zur Erzeugung von elektrischem Strom mittels des mindestens einen photovoltaischen Konverters ist.

2. Medizinprodukt gemäß Anspruch 1, wobei die Sterilverpackung insgesamt oder zumindest in dem den mindestens einen photovoltaischen Konverter bedeckenden Bereich eine Durchlässigkeit für Strahlung der Wellenlänge von 300 nm bis 1100 nm von ≥ 50 % aufweist.

3. Medizinprodukt gemäß Anspruch 1 oder 2, wobei die Sterilverpackung durchlässig für Wasserstoffperoxid, Ethylenoxid, Ozon, Formaldehyd und/oder Peressigsäure ist.

4. Medizinprodukt gemäß einem der Ansprüche 1 - 3, wobei die Sterilverpackung insgesamt oder zumindest in dem den mindestens einen photovoltaischen Konverter bedeckenden Bereich derart durchlässig für Strahlung der Wellenlänge von 300 nm bis 1100 nm ist, dass die auf den mindestens einen photovoltaischen Konverter aufgestrahlte Lichtleistung mindestens 150 mW/cm² beträgt.

5. Medizinprodukt gemäß einem der Ansprüche 1 - 4, wobei der mindestens eine photovoltaische Konverter in die Oberfläche des Medizinproduktes integriert ist.

6. Medizinprodukt gemäß einem der Ansprüche 1 - 5, wobei der mindestens eine photovoltaische Konverter stromleitend und abtrennbar mit dem Medizinprodukt verbunden ist.

7. Medizinprodukt gemäß einem der Ansprüche 1 - 6, wobei die Sterilverpackung zumindest in dem den mindestens einen photovoltaischen Konverter bedeckenden Bereich durch eine entfernbare und nicht lichtdurchlässige Abdeckung versehen ist.

8. Medizinprodukt gemäß einem der Ansprüche 1 - 7, wobei die interne Energieversorgungseinheit zur Versorgung einer Lichtquelle, eines Motors oder eines Impulsgebers dient.

9. Vorrichtung zum Aufladen der internen Energieversorgungseinheit eines Medizinproduktes gemäß einem der Ansprüche 1 - 8, wobei die Vorrichtung eine Wannenform oder Beckenform aufweist und an zumindest einer Innenfläche der Wanne oder des Beckens mindestens eine Lichtquelle zur Erzeugung von durch den mindestens einen photovoltaischen Konverter des Medizinproduktes in elektrischen Strom umwandelbarer Strahlung angeordnet ist und das in der Sterilverpackung befindliche sterilisierte Medizinprodukt formschlüssig in die Wanne oder in das Becken so aufnehmbar ist, dass die mindestens eine Lichtquelle und der mindestens eine photovoltaischen Konverter sich gegenüber liegen.

10. Vorrichtung gemäß Anspruch 9, wobei das in der Sterilverpackung befindliche sterilisierte Medizinprodukt derart formschlüssig in die Wanne oder in das Becken aufnehmbar ist, dass der Abstand zwischen der mindestens einen Lichtquelle und dem mindestens einen photovoltaischen Konverter nicht mehr als 10 mm beträgt.

11. Vorrichtung gemäß Anspruch 9 oder 10, wobei das in der Sterilverpackung befindliche sterilisierte Medizinprodukt derart formschlüssig in die Wanne oder in das Becken aufnehmbar ist, dass der mindestens eine photovoltaische Konverter von der mindestens einen Lichtquelle mit einer Lichtstärke von mindestens 150 mW/cm² bestrahlt wird.

12. Vorrichtung gemäß Anspruch 9, 10 oder 11 mit formschlüssig aufgenommenem Medizinprodukt gemäß eines der Ansprüche 1 - 8.

13. Verfahren zum Aufladen der internen Energieversorgungseinheit eines Medizinproduktes gemäß eines der Ansprüche 1 - 8, wobei der mindestens eine photovoltaische Konverter der Strahlung mindestens einer externen Lichtquelle ausgesetzt wird und die Strahlung der mindestens einen externen Lichtquelle die Sterilverpackung passiert, ohne die Sterilität zu beeinflussen und von dem mindestens einen photovoltaischen Konverter in elektrischen Strom umgewandelt wird, der zum Aufladen der internen Energieversorgungseinheit dient.

14. Verfahren zum Aufladen gemäß Anspruch 13, wobei der mindestens eine photovoltaische Konverter von der mindestens einen Lichtquelle mit einer Lichtstärke von mindesten 150 mW/cm² bestrahlt wird.

15. Verfahren zum Aufladen gemäß Anspruch 13 oder 14, wobei die Sterilverpackung insgesamt oder zumindest in dem den mindestens einen photovoltaischen Konverter bedeckenden Bereich eine mittlere Durchlässigkeit für Strahlung der Wellenlänge von 300 nm bis 1100 nm von ≥ 50 % aufweist.
